# EUROPEAN PATENT SPECIFICATION

(11) **EP 2 889 286 B1**
(45) Date of publication and mention of the grant of the patent: **18.05.2016**
(21) Application number: 13199793.4
(22) Date of filing: 30.12.2013
(51) Int. Cl.: C07C 235/60, A61K 31/616, A61P 29/00

(54) **Pharmaceutically active compound for use as anti-inflammatory agent**
Pharmazeutisch aktive Verbindung zur Verwendung als entzündungshemmendes Mittel
Composé pharmaceutiquement actif destiné à être utilisé comme agent anti-inflammatoire

(43) Date of publication of application: 01.07.2015
(73) Proprietor: Pax Forschung GmbH, 50933 Köln (DE)
(72) Inventor: Ghyczy, Miklós, 50933 Köln (DE)
(74) Representative: von Kreisler Selting Werner - Partnerschaft von Patentanwälten und Rechtsanwälten mbB

(56) References cited:
- WO-A1-98/43676
- US-A- 5 506 224
- US-A1- 2010 041 748
- US-A1- 2013 046 013

## Description

The present invention relates to a medicinally active compound of formula (I) and its use as anti-inflammatory agent. Further, the present invention relates to a method for the preparation of the compound of formula (I) as well as to a pharmaceutical composition comprising a compound of formula (I).

Tumor necrosis factor alpha (TNF-α) is an adipokine involved in systematic inflammation and is a member of a group of cytokines that stimulate the acute phase reaction. It is produced chiefly by activated macrophages (M1), although it can be produced by many other cell types such as CD4+ lymphocytes, NK cells and neurons.

The primary role of TNF-α is in the regulation of immune cells. TNF-α, being an endogenous pyrogen, is able to induce fever, apoptotic cell death, cachexia, inflammation and to inhibit tumorigenesis and viral replication and respond to sepsis via IL1IL6 producing cells. Dysregulation of TNF-α production has been implicated in a variety of human diseases including Alzheimer's disease, cancer, major depression and inflammatory bowel disease (IBD). While still controversial, studies of depression and IBD are currently being linked by TNF-α levels. Recombinant TNF-α can be produced ectopically in the setting of malignancy and parallels parathyroid hormone both in causing secondary hypercalcemia and in the cancers with which excessive production is associated.

Therefore, in order to provide effective treatment of the above mentioned diseases there is a high demand for TNF-α inhibitors. A TNF-α inhibitor is a pharmaceutical drug that suppresses response to TNF-α. Inhibition of TNF-α can, for example, be achieved with a monoclonal antibody or with a circulating receptor fusion protein such as etanercept.

Etanercept, which is commonly known under its trade name of Enbrel is made from the combination of two naturally occurring soluble human 75-kDa TNF reactors linked to an Fc portion of an IgG1. The effect is an artificially engineered dimeric fusion protein. In the USA the FDA has licensed Enbrel for moderate to severe rheumatoid arthritis, moderate to severe polyarticular juvenile idiopathic arthritis (JIA), psoriatic arthritis, ankylosing spondylitis (AS) and moderate to severe plaque psoriasis. However, patients treated with Enbrel also suffered severe side effects, including infections or worsening of infections the patient already had; reactivation of hepatitis B; nervous system problems, such as multiple sclerosis, seizures or inflammation of the nerves of the eyes; blood problems; heart failure; psoriasis; allergic reactions; and autoimmune reactions, including lupus-like syndrome and autoimmune hepatitis.

Infliximab, commonly known under its trade name Remicade, is a TNF-α inhibitor that is approved for the treatment of psoriasis, Crohn's disease, ankylosing spondylitis, psoriatic arthritis, rheumatoid arthritis and ulcerative colitis in the US. Remicade is an artificial antibody which was originally developed in mice as a mouse antibody. Because humans have immune reactions to mouse proteins, the mouse common domains were replaced with similar human antibody domains. Due to the combination of mouse and human antibody amino acid sequences, it is called a chimeric monoclonal antibody. However, Infliximab has adverse effects common to drugs in the class of immunosuppressants, including serious and sometimes fatal blood disorders; serious infections; lymphoma and solid tissue cancers; serious liver injury; reactivation of hepatitis B; reactivation of tuberculosis; lethal hepatosplenic T-cell lymphoma; drug-induced lupus; demyelinating central nervous system disorders; psoriasis and psoriasiform skin lesions and new-onset vitiligo.

US 5,506,224 describes N-acyl-derivatives of hydroxyamines which are suitable for the therapeutic treatment of pathologies characterized by degranulation of mast cells caused by a neurogenic and/ or immunogenic hyperstimulation.

Due to the fact that the known TNF-α inhibitors presently on the market are accompanied by severe adverse effects, in part due to the fact that they are proteins, as well as due to their high costs, there is still a high demand for TNF-α inhibitors that do not suffer the above-mentioned draw backs and that are able to overcome the disadvantages of the known antibody TNF-α inhibitors.

It is therefore an object of the present invention to provide a medicinally active compound, preferably an anti-inflammatory agent that acts as a TNF-α inhibitor as well as a pharmaceutical composition comprising the same for use in the treatment of inflammatory processes, especially inflammatory bowel diseases.

The object of the present invention is solved by the provision of a compound of formula (I) which surprisingly shows activity in the treatment of inflammatory processes.

One object of the present invention is therefore a compound of formula (I):

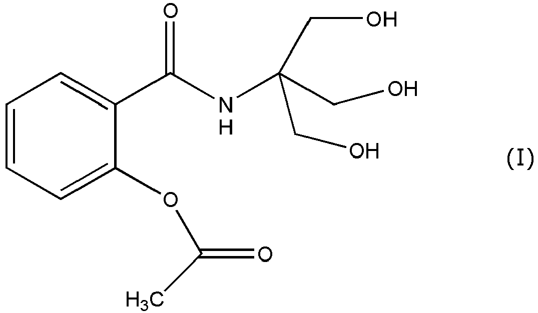

In a preferred embodiment of the present invention the compound of formula (I) is present as a salt. Preferably, the counter ion is a pharmaceutically acceptable counter ion of organic or inorganic nature, such as acetate, palmitate, citrate, sulfate, carbonate and hydrochloride.

Preferably the compound of formula (I) is for use in the treatment and/or prophylaxis of diseases associated with inflammatory processes of the human or animal body, in particular diseases selected from the group consisting of inflammatory bowel disease, psoriasis, Crohn's disease, ankylosing spondylitis, psoriatic arthritis, rheumatoid arthritis and ulcerative colitis.

It was surprisingly found that the compound of formula (I) possess a high medicinal activity, especially when employed in the treatment of inflammatory processes. A further object of the present invention is therefore an anti-inflammatory agent of a compound of formula (I) as defined in the present invention.

As there is still a high demand for effective and affordable drugs, another object of the present invention is a pharmaceutical composition comprising a compound of formula (I)

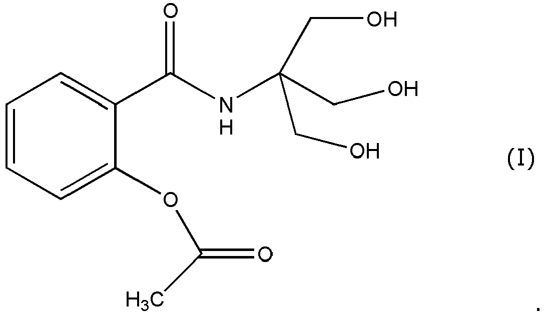

In a further preferred embodiment the pharmaceutical composition according to the invention comprises a compound of formula (I) or a pharmaceutical acceptable salt thereof.

Preferably the pharmaceutical composition according to the invention comprises the compound of formula (I), or a pharmaceutical acceptable salt thereof, in a pharmaceutically effective amount, preferably ranging from 0.001 to 10% by weight, based on the total weight of the pharmaceutical composition. Preferably the amount of the compound of formula (I), or a pharmaceutical acceptable salt thereof, is high enough to be pharmaceutically effective.

A surprising activity of the pharmaceutical composition according to the invention in the inhibition of TNF-α has been found. An increase of TNF-α is associated with inflammatory processes, especially inflammatory diseases, such as psoriasis, arthritis and inflammatory bowel diseases.

Further, it has surprisingly been found that the compound of the invention reduces the peroxidase activity which is an advantage compared to other acetyl salicyl acid derivatives such as O-acetylsalicyl derivatives selected from the group consisting of O-acetylsalicyl amino carbohydrates, O-acetylsalicyl amino acids and O-acetylsalicyl peptides.

Additionally, it has surprisingly been found that the compound of the invention reduces the concentration of the high-mobility group protein B1 in the plasma which makes this compound an interesting candidate in the treatment of age-related diseases. The compound of the present invention positively influences the redox balance.

Therefore, in a further embodiment of the present invention the compound of the present invention can be used in the treatment or prophylaxis of pathological aging, in particular selected from obesity, sarcopenia, metabolic syndrome, dementia, cancer, atherosclerosis and osteoporosis.

A further aspect of the invention is an embodiment wherein the pharmaceutical composition is for use in the treatment and prophylaxis of inflammation. Preferably the pharmaceutical composition is for use in the treatment of mammals. Further preferred is an embodiment wherein the pharmaceutical composition according to the invention is used in the treatment of pain and fever.

Any medicinally active compound or composition has to be used with all due care, in particular overdoses are to be avoided under all circumstances. Therefore, the pharmaceutical composition is preferably administered in a dosage ranging from 0.001 to 0.3 g per kg body weight and day. Further preferred is an embodiment wherein the pharmaceutical composition for use in the treatment and prophylaxis of inflammation is administered in a dosage ranging from 0.001 to 0.3 g per kg bodyweight and day.

In specific embodiments the dosage may vary depending on the condition of the patient and the disease to be treated. A further preferred dosage is ranging from 0.005 to 0.15, more preferably 0.01 to 0.1 or 0.05 to 0.5 g per kg body weight and day.

In particular, an embodiment of the present invention is preferred wherein the pharmaceutical composition according to the invention is for use in the treatment or prophylaxis of diseases selected from the group consisting of
a) psoriasis,
b) Crohn's disease,
c) ankylosing spondylitis,
d) psoriatic arthritis,
e) rheumatoid arthritis, and
f) ulcerative colitis.

Further preferred is an embodiment of the pharmaceutical composition according to the invention for use in the treatment or prophylaxis of inflammatory bowel diseases.

Any drug administered to a mammal, especially a human, suffering from diseases, should have high bioavailability in order to guarantee a treatment as effective and fast as possible. At the same time, the stress and inconveniences suffered by the sick person should be kept to a minimum.

Therefore, in a preferred embodiment, the pharmaceutical composition according to the invention is administered orally and/or parenterally. In an alternative preferred embodiment, the pharmaceutical composition according to the invention is applied topically to the patient, preferably in the form of an oil-in-water emulsion or a water-in-oil emulsion. In a still alternatively preferred embodiment the pharmaceutical composition for use according to the invention is administered in the form of tablets or capsules.

The compound of the present invention may also be combined with other medicinally active ingredients. In a further embodiment the pharmaceutical composition of the present invention may be formulated as solution, gel, lotion, cream, ointment, shampoo, spray, stick, powder, mouth rinse or wash, vaginal gel or preparation, or other form acceptable for use on skin, nail, hair, oral mucosa, vaginal or and mucosa, mouth or gums.

The pharmaceutical composition may be administered topically, orally, parenterally. Preferably, the composition is administered by injection, infusion or oral intake.

The pharmaceutical composition according to the invention may additionally comprise further components. The nature of these additional components may depend on the form of administration that is used to administer the pharmaceutical composition according to the invention to the patient. These components may be galenic additives known to the person skilled in the art, such as tonicity agents, pharmaceutically acceptable solvents, disingrediants, lubricants, glidants, solubilizers, stabilizers, buffers, tonicity modifiers, bulking agents, viscosity enhancer/reducers, surfactants, chelating agents, adjuvants and the like. The pharmaceutical composition according to the invention may, e.g. comprise one or more filler, such as glucose, mannitol or sorbitol, one or more binders, such as MCC (microcrystalline cellulose), starch, cellulose ether or gelatin, and/or one or more exploder such as potato starch, corn starch, polyvinyl pyrrolidone (PVP), carbopol and magnesia peroxide. Further, the pharmaceutical composition according to the invention may comprise. The additives of the pharmaceutical composition are preferably chosen in accordance with the form of administration.

A further embodiment of the present invention is a method of preparation of a compound of formula (I). The method of preparation according to the invention comprises the following step:
reacting a compound of formula (II) 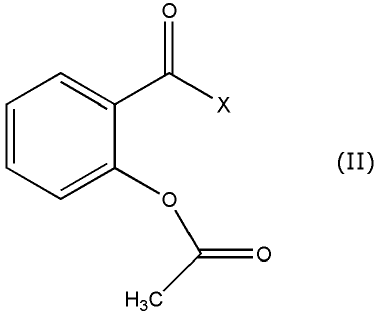 wherein X is a leaving group;
with a compound of formula (III) 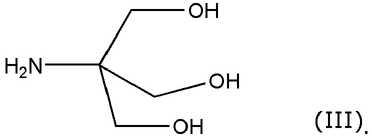 under formation of a compound of formula (I).

Especially preferred is an embodiment wherein X is selected from the group consisting of Halogen, -OH and -N₃. In a particular preferred embodiment X is - Cl.

In a further preferred embodiment the method according to the invention comprises the following steps:
i) providing a compound of formula (IV) 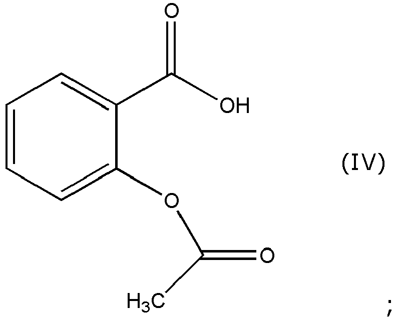
ii) activating the acid group of the compound of formula (IV);
iii) converting the activated form of the compound of formula (IV) with a compound of formula (III) 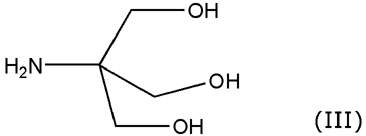 under formation of a compound of formula (I).

Preferably the activation of the acid group (-C(O)OH) of the compound of formula (I) is carried out in the presence of a base.

Further preferred is an embodiment of the method according to the invention wherein steps i) and ii) are carried out in an organic solvent, preferably an aprotic organic solvent which is essentially free of water and possesses sufficient solubility properties to dissolve the reaction components. Essentially free of water in the context of the present invention refers to solvents that contain water in an amount of less than 500 ppm, preferably less than 250 ppm and especially preferred in a range from 0.001 ppm to 100 ppm, wherein the ppm are based on weight.

In an especially preferred embodiment the method according to the invention is carried out as a one-pot-reaction, i.e. steps i) to iii) are carried out in one reaction vessel, without isolation of the product of step ii).

Further preferred is an embodiment wherein step ii) of the method according to the invention is carried out at a temperature ranging from -25 °C to -5 °C, preferably ranging from -20 °C to -10 °C.

In a further preferred embodiment, step iii) of the method according to the invention is carried out at a temperature ranging from -25 °C to 30 °C, preferably ranging from -15 °C to 25 °C.

The present invention is further described in more detail in the following examples which are not to be understood to limit the present invention in any form.

### Examples:

### a) Preparation of a compound of formula (I):

2.88 g acetyl salicylic acid was dissolved in 150 ml abs. tetrahydrofurane and cooled to -15 °C. Under stirring 2.1 ml of isobutylchloroformate and 2.23 ml triethylamine were added. After 25 minutes of stirring at -15 °C, 1.94 g of tris hydroxymethyl amino methane was added. The reaction mixture was stirred at 0 °C for one hour. Stirring was continued at room temperature overnight. The reaction mixture was filtered, the solvent evaporated and the resulting crystalline material was washed with diethylether - hexane, rendering the pure product in form of the compound of formula (I).
Yield: 2.34 g (52%)
GM12 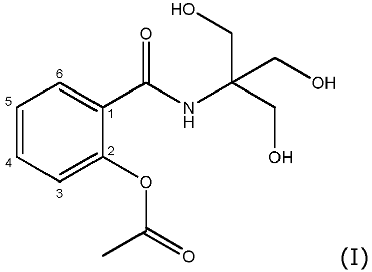
¹H NMR (600 MHz, DMSO): δ= 2.29 (s, 3H, CH₃), 3.65 (d, J = 4.90 Hz, 6H, 3 x *CH₂*-OH), 4.77 (t, J = 4.90 Hz, 3H, CH₂-*OH*), 7.19 (d, J = 7.53 Hz, 1H, H-3), 7.26 (s, 1H, NH), 7.34 (t, J = 7.53 Hz, 1H, H-5), 7.52( t, J = 7.53 Hz, 1H, H-4), 7.69 (d, J = 7.53 Hz, 1H, H-6);
¹³C NMR (150 MHz, DMSO): δ=21.3 (CH₃), 60.6 (*CH₂*-OH), 63.0 (C-CH₂-OH), 123.8 (C-3), 126.4 (C-5), 129.7 (C-1), 130.2 (C-6), 131.9 (C-4), 147.9 (C-2), 165.6 (C=O-NH), 169.4 (O-C=O).

The purity and identity was confirmed by electrospray MS and HPLC investigations.

### b) Clinical tests

The medicinal activity of the compound according to the invention and the pharmaceutical composition according to the invention was tested in a series of experiments performed on rats. The experiments were performed on male Sprague-Dawley rats (280-320 g) housed in plastic cages in a thermoneutral environment (21 °C ± 2 °C) with a 12 h dark-light cycle. The animals, fed on a normal diet with tap water *ad libitum,* were randomly allocated into experimental groups according to the feeding protocols.

Further, experiments were performed on mice held under the same conditions.

### Induction of colitis

Colonic inflammation was induced by intracolonic administration of TNBS (2,4,6-trinitrobenzenesulfonic acid) (40 mg/kg in 0.25 ml of 25% aqueous ethanol) through an 8-cm-long soft plastic catheter under transient diethylether anesthesia. In the sham-operated groups, only the vehicle for TNBS was administered. The animals were deprived of food, but not water, for 12 h before the enemas.

### Surgical preparation

The animals were anesthetized with sodium pentobarbital (50 mg/kg body weight i.p.) 1 or 3 days after the enema and placed in a supine position on a heating pad. Tracheostomy was performed to facilitate spontaneous breathing, and the right jugular vein was cannulated with PE-50 tubing for fluid administration and Ringer's lactate infusion (10 ml/kg per hour) during the experiments. A thermistor-tip catheter (PTH-01; Experimetria Ltd, Budapest, Hungary) was positioned into the ascending aorta through the right common carotid artery to measure the CO by thermodilution technique. The right femoral artery was cannulated with PE-40 tubing for mean arterial pressure (MAP) and heart rate (HR) measurements.

### Hemodynamic measurements

Pressure signals (BPR-02 transducer, Experimetria Ldt, Budapest, Hungary) were measured continuously and registered with a computerized data-acquisition system (Experimetria Ldt). Cardiac output was detected by a thermodilution technique, using a SPEL Advanced Cardosys 1.4 computer (Experimetria Ldt). Gases in arterial blood samples were measured with a blood gas analyzer (AVL Compact 2, Graz, Austria).

### Preparation of colon biopsies

Colon biopsies kept on ice were homogenized in phosphate buffer (pH 7.4) containing 50 mM Tris-HCl (Reanal, Budapest, Hungary), 0.1 mM EDTA, 0.5 mM dithiothreitol, 1 mM phenylmethylsulfonyl fluoride, 10 µg/ml soybean trypsin inhibitor, and 10 µg/ml leupeptin (Sigma Aldrich GmbH, Munich, Germany). The homogenate was centrifuged at 4 °C for 20 minutes at 24,000 g, and the supernatant was loaded into centrifugal tubes (Amicon Centricon-100; 100,000-molecular-weight cutoff ultrafilter, Millipore Corporation, Bedford, MA). The acitivity of myeloperoxidase (MPO) was measured on the pellet of the homogenate.

### Plasma nitrite/nitrate measurements

The levels of plasma nitrite/nitrate (NOₓ), stable end products of NO were measured by the Griess reaction. This assay depends on the reduction of nitrate to nitrite, which is then converted into a colored azo dye compound that is detected spectrophotometrically at 540 nm.

### Measurement of plasma TNF-α

Blood samples (0.5 ml) were taken from the inferior caval vein into precooled, heparinized (100 U/ml) polypropylene tubes, centrifuged at 1,000 *g* at 4 °C for 30 minutes and then stored at -70 °C until assay. Plasma TNF-α concentrations were determined in duplicate by means of a commercially available enzyme-linked immunosorbent assay (Quantikine ultrasensitive enzyme-linked immunosorbent assay kit for rat TNF-α; Biomedica Hungaria Kft, Budapest, Hungary). The minimum detectable level was less than 5 pg/ml, and the inter-assay and intra-assay coefficients of variation were less than 10%.

### Xanthine oxidoreductase enzyme acitivity

The XOR activity was determined in the ultrafiltered, concentrated supernatant by fluorometric kinetic assay based on the conversion of pterine to isoxanthopterine in the presence (total XOR) or absence (xanthine oxidase activity) of the electron acceptor methylene blue.

### Tissue MPO activity

The activity of MPO as a marker of tissue leukocyte infiltration was measured on the pellet of the homogenate. Briefly, the pellet was resuspended in K₃PO₄ buffer (0.05 M; pH 6.0) containing 0.5% hexa-1,6-*bis*-decyltriethylammonium bromide. After three repeated freeze-thaw procedures, the material was centrifuged at 4 °C for 20 minutes at 24,000 g, and the supernatant was used for MPO determination. Subsequently, 0.15 ml of 3,3',5,5'-tetramethylbenzidine (dissolved in dimethyl sulfoxide, 1.6 mM) and 0.75 ml of hydrogen peroxide (dissolved in K₃PO₄ buffer, 0.6 mM) were added to 0.1 ml of the sample. The reaction led to the hydrogen peroxide-dependent oxidation of tetramethylbenzidine, which could be detected spectrophotometrically at 450 nm (UV-1601 spetrophotometer; Shimadzu, Kyoto, Japan). Myeloperoxidase activities were measured at 37 °C; the reaction was stopped after 5 minutes by the addition of 0.2 ml of H₂SO₄ (2 M), and the resulting data were referred to the protein content.

### Intravital videomicroscopy measurements

The intravital orthogonal polarization spectral imaging technique (Cytosan A/R; Cytometrics, Philadelphia, Pa) was used for continous visualization of the microcirculation of the colon serosa. This technique uses reflected polarized light at the wave length of the isobestic point of oxyhemoglobin and deoxyhemoglobin (548 nm). Because polarization is preserved in reflection, only photons scattered from a depth of 200 to 300 µm contribute to the image formation. A 10x objective was introduced into the intestinal lumen, and the microscopic images were recorded with an S-VHS video recorder (Panasonic AG-TL 700; Matsushita Electric Ind Co Ltd, Osaka, Japan). Microcirculatory evaluation was performed off-line by frame-to-frame analysis of the video taped images. The changes in capillary red blood cell velocity (RBCV, µm/s) in the postcapillary venules were determined in three separate fields by means of a computer-assisted image analysis system (IVM Pictron, Budapest, Hungary). All microcirculatory evaluations were performed by the same investigator.

### Determination of High-mobility group protein B1

High mobility group box protein 1 (HMGB-1) measurements in plasma:
Blood samples were drawn into chilled polypropylene tubes containing EDTA (1 mg ml-1) and were centrifuged at 1200g for 10 min at 4 oC. The plasma samples were then collected and stored at -70 oC until assay. Plasma concentration of HMGB-1 was measured by a commercially available HMGB-1 ELISA kit (Shino-Test Corporation, Kanagawa, Japan).

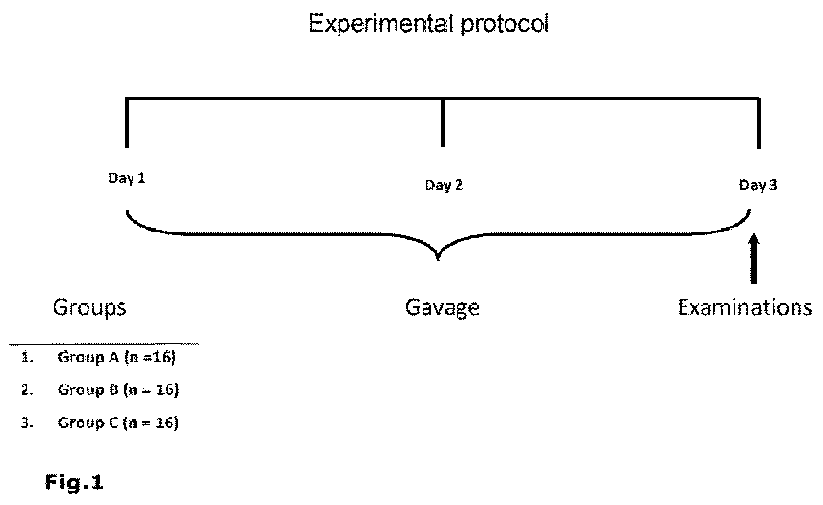

### Experimental protocol (Figure 1)

First experiments regarding the effect of the compound of formula (I) and a pharmaceutical composition comprising the compound of formula (I) (clear aqueous solution of compound of formula (I) at pH 4.8), respectively, on gastric injury without inflammation were conducted on mice. 48 animals were randomly allocated into three groups. Group A (n = 16) served as sham-operated controls. Samples of all animals were drawn on day 1 of the clinical tests. On day 2 the animals of group B (n = 16) were force-fed acetylsalicylic acid (ASS) (1.11 mmol/kg body weight). Group C (n = 16) was force-fed 1.11 mmol/kg body weight of the compound according to the invention. Group A only received the respective solvent (25% ethanol in water). One day after the gavage, additional samples were drawn. Fluorescence confocal endomicroscopy was performed to examine the microvasculature and morphologic changes of the mucosa of the distal colon, and full-thickness tissue samples and venous blood samples were taken to determine the biochemical changes in the colon and plasma. The effects were determined by way of measurement of body weight, blood gas analysation and biopsy. ASS was chosen as a comparative example due to its structural similarity and its known activity as anti-inflammatory agent.
Figure 1 shows the experimental protocol I for groups A, B and C.

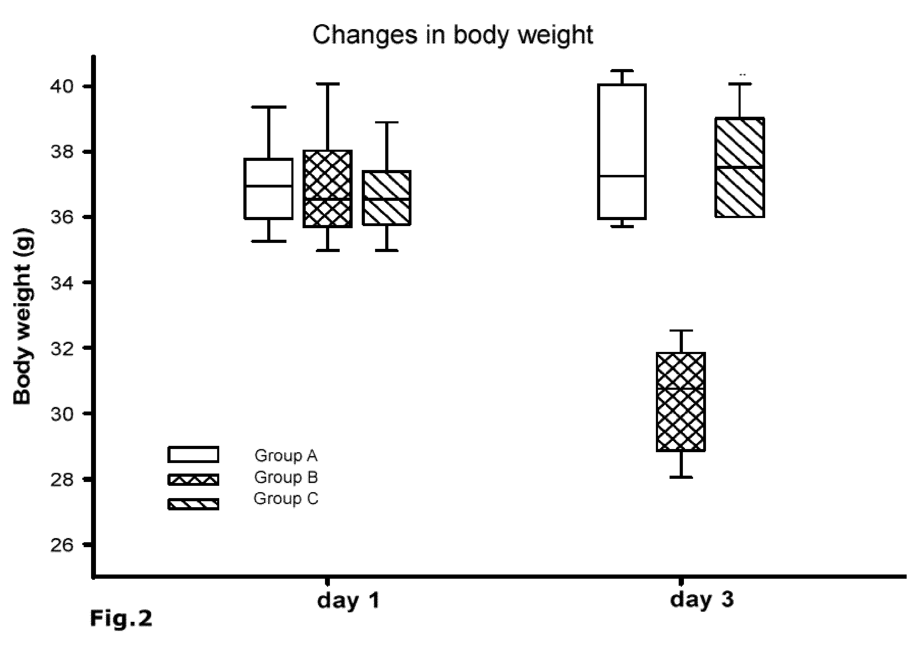

Figure 2 shows the change in body weight of the test animals with gastric injury without inflammation before and after the administration of ASS (group B) and the compound according to the invention (group C). Group A served as sham-operated controls. As can be seen from the data shown in Figure 2 the test animals suffered a severe loss of body weight within one day when administered ASS. In comparison, animals which were treated with the compound according to the invention only showed a minor loss in body weight compared to the animals that did not receive any pharmaceutically active compound. Further, the data clearly show that the compound of the invention does not cause additional stress and shows no adverse effects that would be expressed by loss of body weight.
Figure 3 shows the survival rate of the animals in group A, B and C.
Figure 4 shows the red blood cell velocity (RBCV) in the gastric serosa of the animals of group A, B and C.

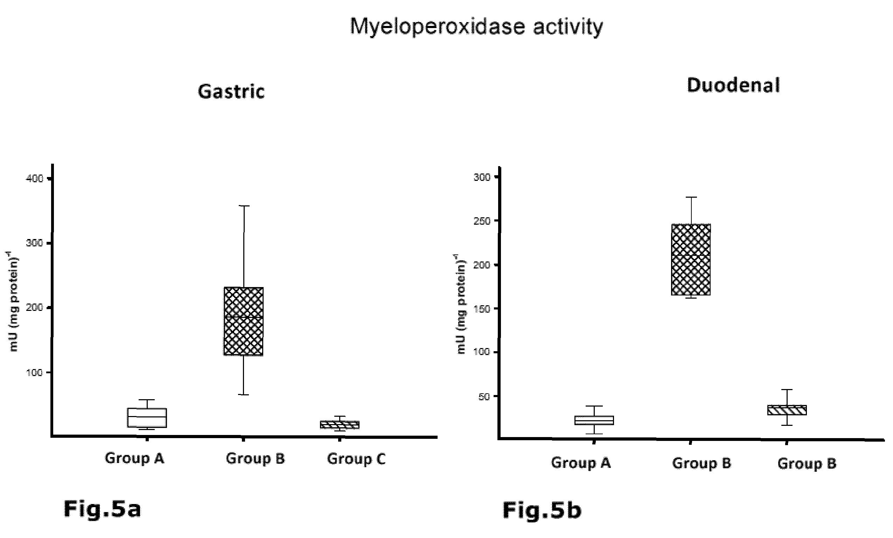

Figures 5a and 5b show the results of the determination of myeloperoxidase (MPO) activity one day after the treatment of the test animals. Figure 5a reflects the data obtained from gastric tissue and Figure 5b reflects the data obtained from duodenal tissue of the animals. As can be seen, the MPO activity of the animals treated with ASS (group B) was increased in comparison to those of the control group (group A) and those which were treated with the compound according to the invention (group C). Since MPO is an enzyme associated with inflammatory processes, it can be concluded that the treatment of mice with gastric injury with the compound according to the invention does not further enhance the inflammatory processes of the body, as is the case with ASS.
Figures 6a and 6b show the results obtained by determining the activity of xanthine oxidase enzyme in gastric and duodenal tissue. Whereas animals treated with ASS showed an increased activity (group B), no such increase could be determined by the animals treated with the compound according to the invention (group C) in comparison to the control animals of group A. A high activity of xanthine oxidase enzyme may lead to an increased production of urea, which is associated with gout, and liver damage.
Figures 7a and 7b show the results of the measurement of the nitrite/nitrate level in the gastric tissue (Fig. 7a) and the duodenal tissue (Fig. 7b) of the test animals. Animals treated with ASS (group B) show a significant difference in the nitrite/nitrate level in comparison to animals of the control group A or animals of group C which were treated with the compound according to the invention.
Figurea 8 and 9 show the macroscopic damage of the gastric mucosa of the animals of group A, B and C. For group a damage of the mucosa can be identified.
Figure 10 shows the macroscopic analysis of the mucosa on basis of a score systems wherein the following criteria are applied and equally writhed:
   1. Size of bleeding areas (score: 0-2)
   2. Number of bleeding areas (score: 0-3)
   3. Extension of ulcer (score: 0-2)

The average of the three criteria is calculated for the animals of each group, wherein "0" means for criteria 1 small or no bleeding area, for criteria 2 no bleeding area and for criteria 3 no ulcer.

The higher the number the more severe the damage.

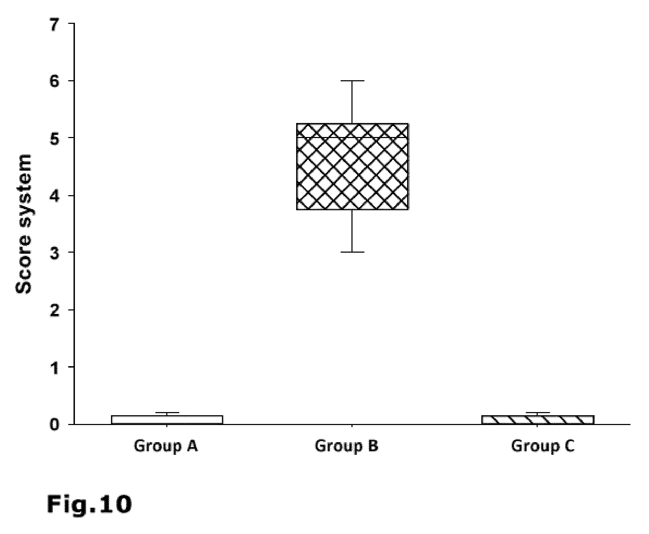

Figure 10 shows that the animals of group B have a significant damage of the mucosa compared to the animals of group A and C.

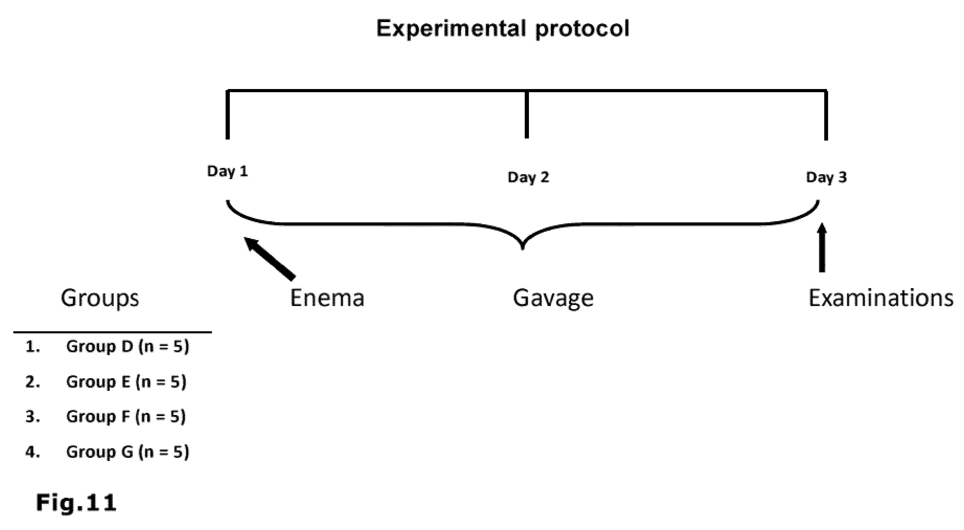

### Experimental Protocol (Figure 11)

The above-described clinical studies were repeated with animals having colitis which was induced as described above. 20 mice were randomly divided into four groups, wherein group D served as sham-operated control, having no colitis, group E served as second control having colitis but receiving no further treatment, group F having colitis and being treated with 1.11 mmol/kg body weight of ASS and group G having colitis and being treated with 1.11 mmol/kg of the compound according to the invention. One day after the animals received the enema with TNBS (40 mg/kg i.c.), wherein the animals of group D only received the respective solvent, the animals of group F and G were treated with ASS and the compound according to the invention, respectively. The other animals only received the respective solvent again. Two days after the enema the effects of the treatment were determined by measurement of body weight, blood gas analysis and biopsy. The animals were anesthetized two days after colitis induction. Surgery was performed to allow registration of the hemodynamic parameters at 1h intervals for 6 h (cardiac output [CO] data were measured only at the end of the experiments). Intravital videomicroscopy was performed at the end of the experiments to visualize the serosal microcirculation 3 cm distal from the cecum. In addition, fluorescence confocal endomicroscopy was performed to examine the microvasculature and morphologic changes of the mucosa of the distal colon, and full-thickness tissue samples and venous blood samples were taken to determine the biochemical changes in the colon and plasma.

Figure 11 shows the experimental protocol for group D, E, F and G.

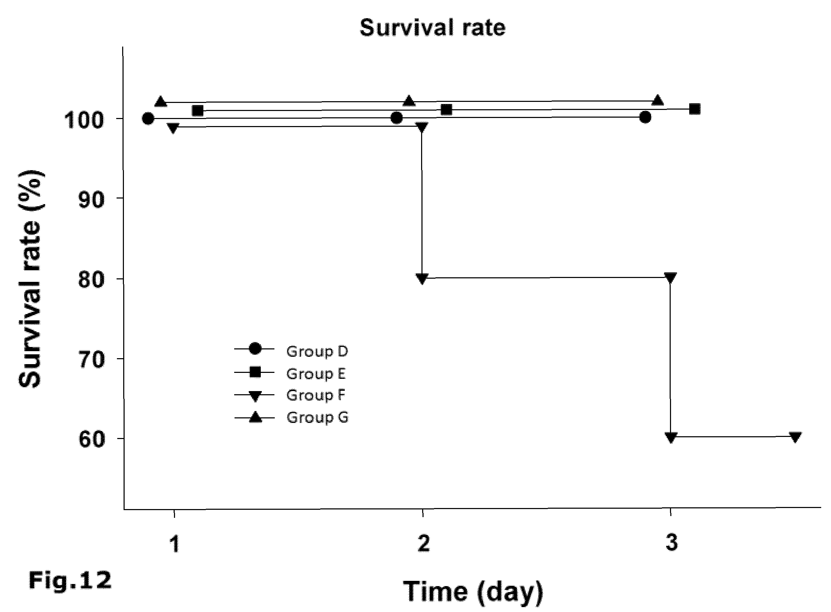

Figure 12 shows the survival rate of the mice in group D, E, F and G.

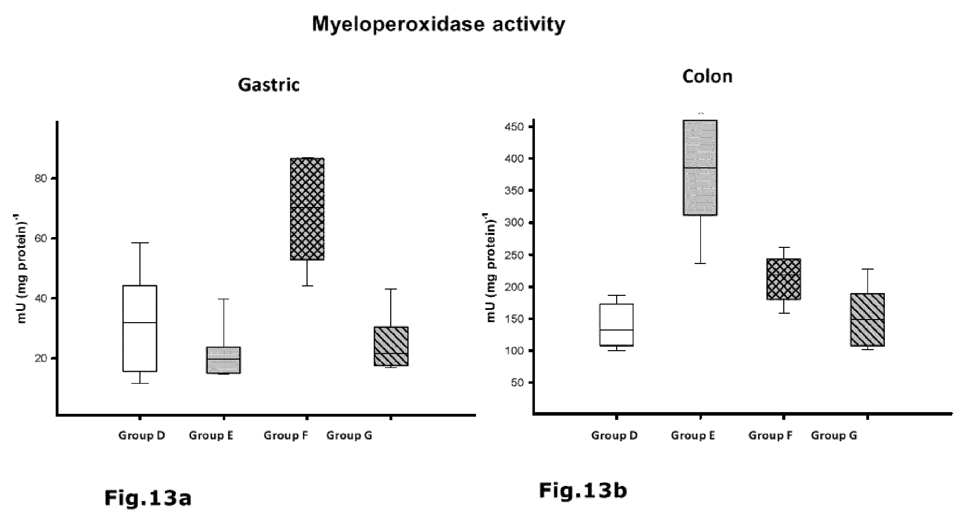

Figures 13a and 13b depict the myeloperoxidase enzyme activity of gastric tissue (Figure 13a) and colon tissue (Figure 13b) determined 3 days after induction of the colitis and one day after treatment. Again, the positive effect of the compound according to the invention (group G) can be seen, resulting in a decrease of activity when compared to animals having colitis without further treatment (group E) and those having colitis and having been treated with ASS (group F).

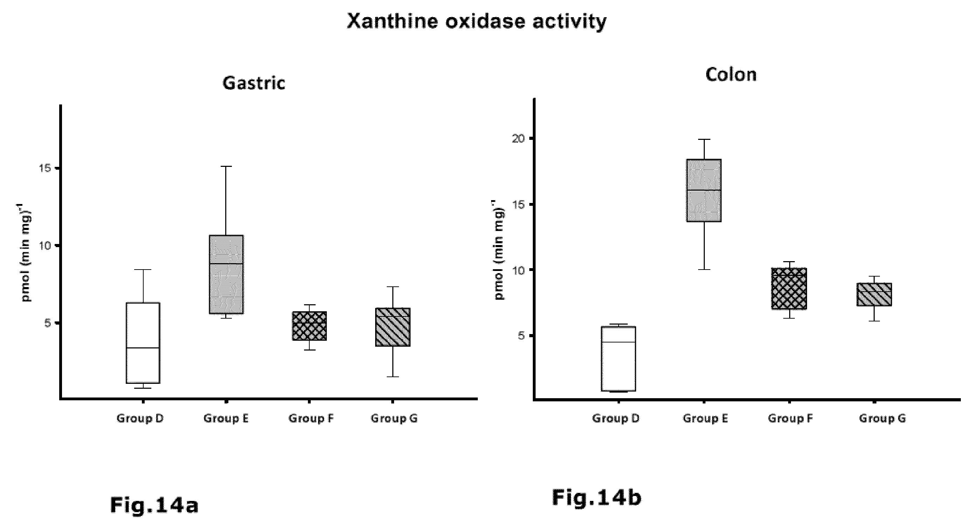

Figures 14a and 14b show the results obtained by determining the activity of xanthine oxidase enzyme in gastric and colon tissue (Figures 14a and 14b, respectively). Whereas animals treated with ASS (group F) show only a slight decrease in activity when compared to those receiving no medicinal treatment (group E), animals of group G, having been treated with the compound according to the invention, showed an enzyme activity comparable to that of healthy animals, represented by group D, in both cases.

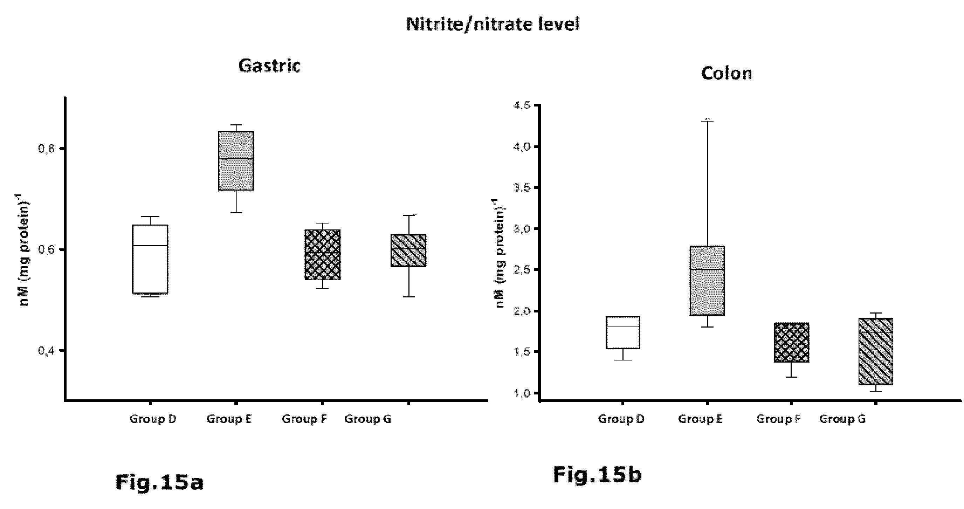

Figures 15a and 15b show the results of the measurement of the nitrite/nitrate level in the gastric tissue (Figure 15a) and the colon tissue (Figure 15b) of the test animals. Animals treated with the compound according to the invention (group G) show nitrite/nitrate levels comparable to those of healthy animals despite having colitis.

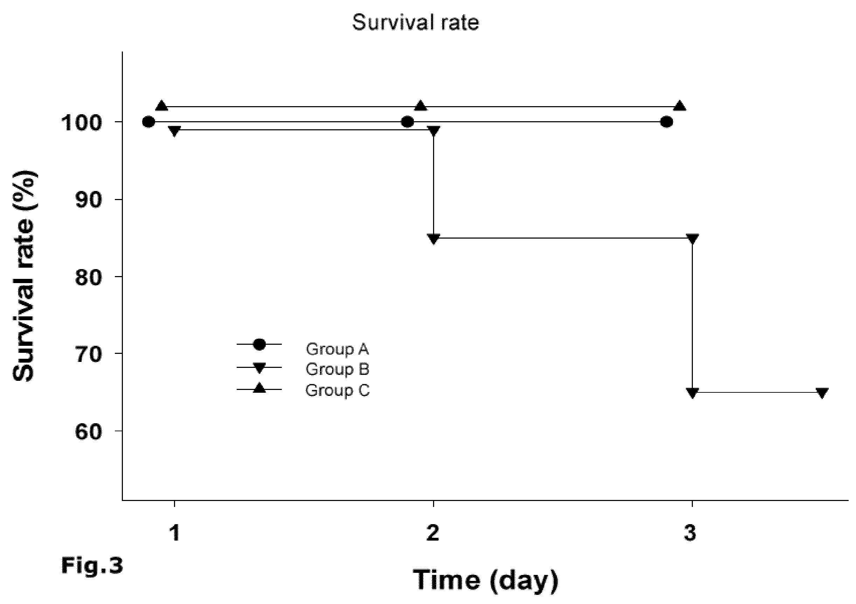

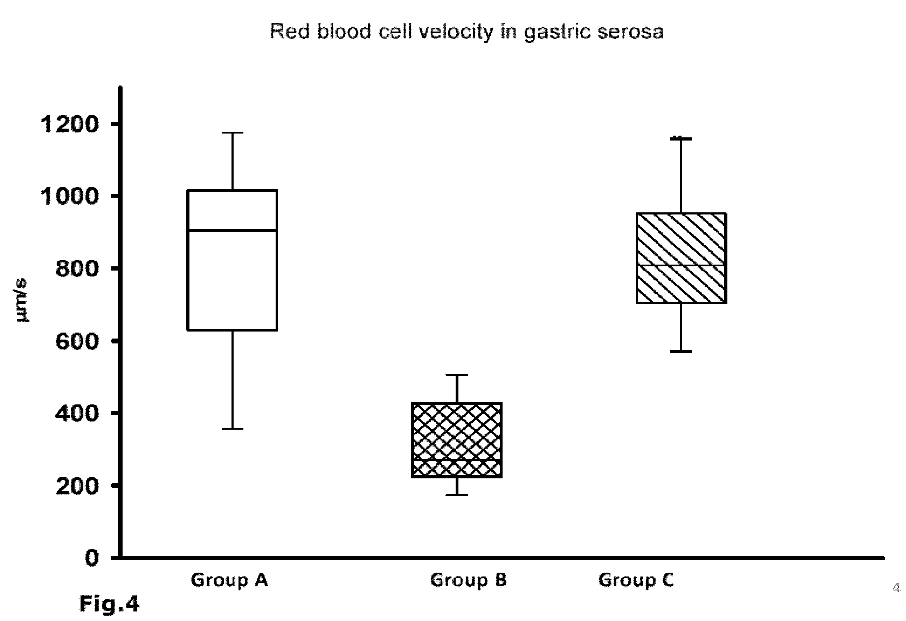

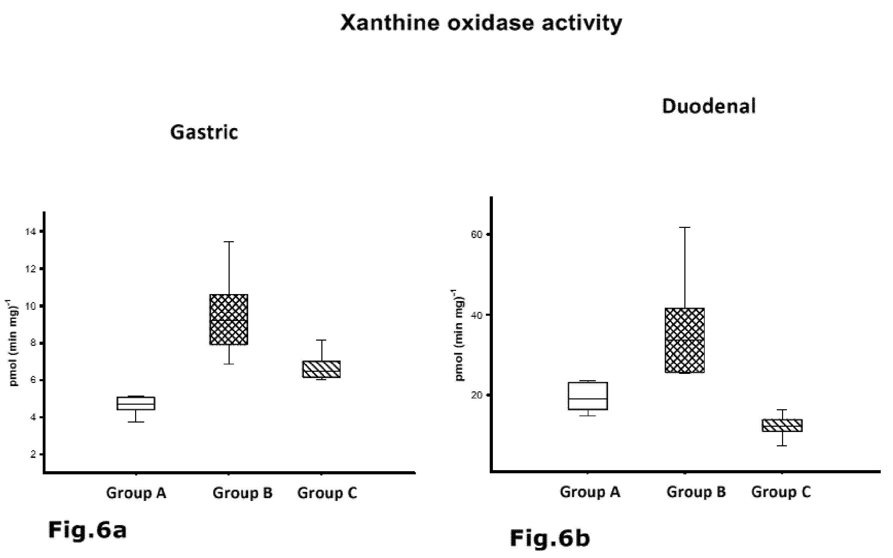

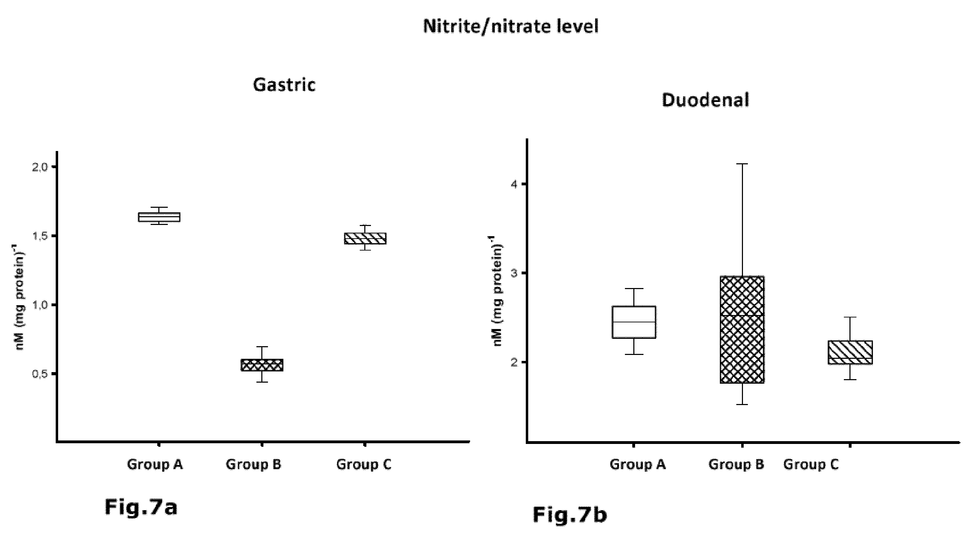

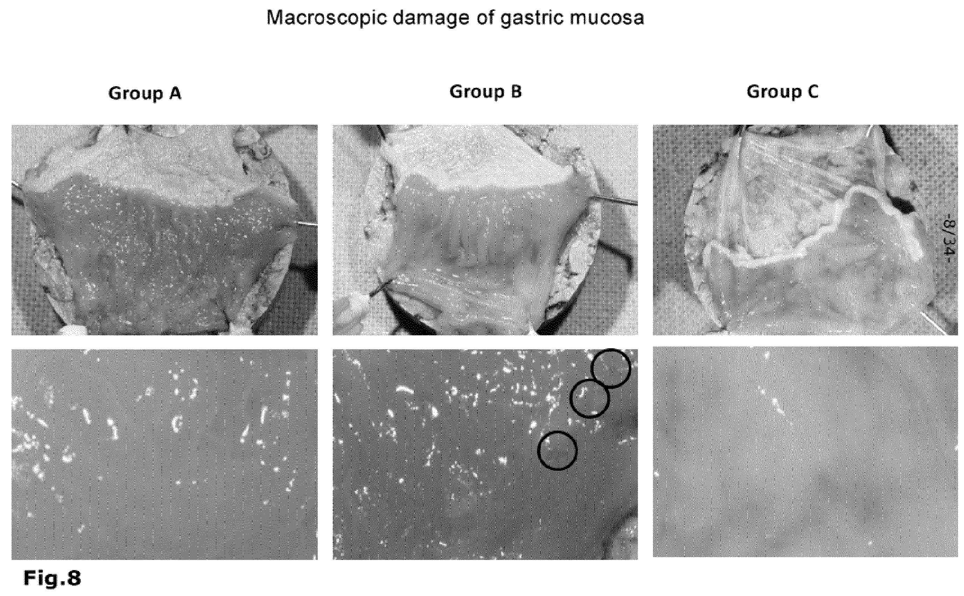

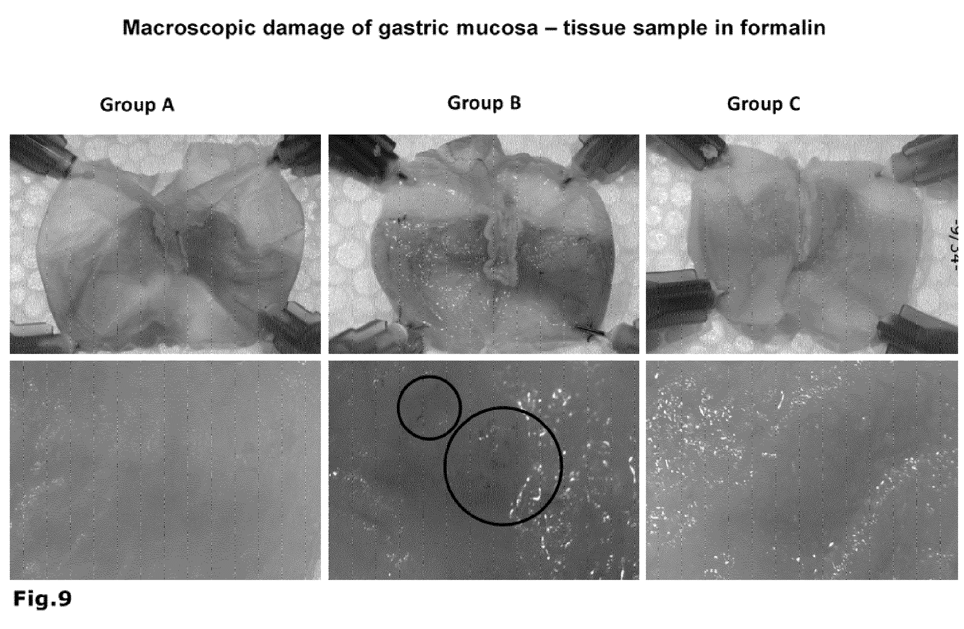

As can be seen from Figures 1 to 15, on the mouse model the compound according to the invention shows a high medicinal activity, especially as TNF-α inhibitor and in the treatment of inflammatory bowel diseases such as colitis. Further, test animals treated with the compound of formula (I) did not suffer the adverse effects observed in the treatment with ASS. Further, with the compound of the invention the damage of the mucosa is reduced and myeloperoxidase activity and xanthine oxidase activity can be reduced.

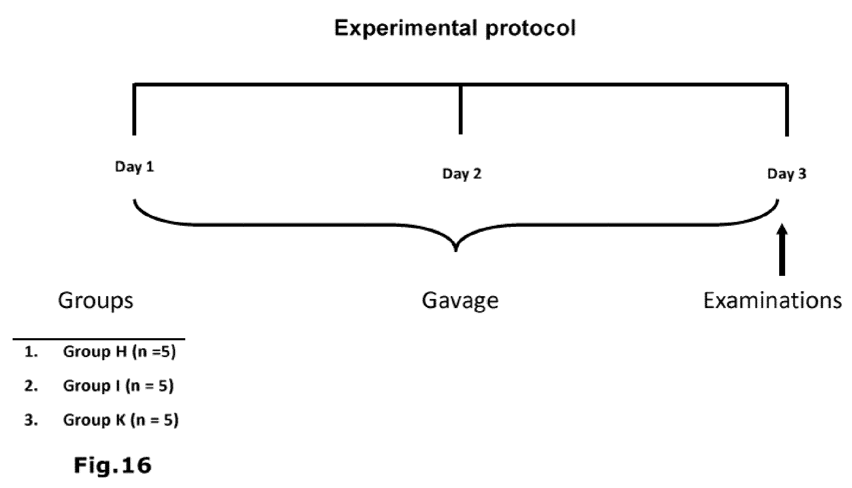

### Experimental protocol (Figure 16)

Further experiments regarding the effect of a compound of formula (I) and a pharmaceutical composition comprising a compound of formula (I), respectively, on gastric injury without inflammation were conducted on rats. 15 animals were randomly allocated into three groups. Group H (n = 5) served as sham-operated controls. Samples of all animals were drawn on day 1 of the clinical tests. On day 2 the animals of group I (n = 5) were force-fed acetylsalicylic acid (ASS) (1.11 mmol/kg body weight). Group K (n = 5) was force-fed 1.11 mmol/kg body weight of the compound according to the invention. Group H only received the respective solvent (25% ethanol in water). One day after the gavage, additional samples were drawn. Fluorescence confocal endomicroscopy was performed to examine the microvasculature and morphologic changes of the mucosa of the distal colon, and full-thickness tissue samples and venous blood samples were taken to determine the biochemical changes in the colon and plasma. The effects were determined by way of measurement of body weight, blood gas analysation and biopsy. ASS was chosen as a comparative example due to its structural similarity and its known activity as anti-inflammatory agent.

Figure 16 shows the experimental protocol on a rat model for groups H, I and K.

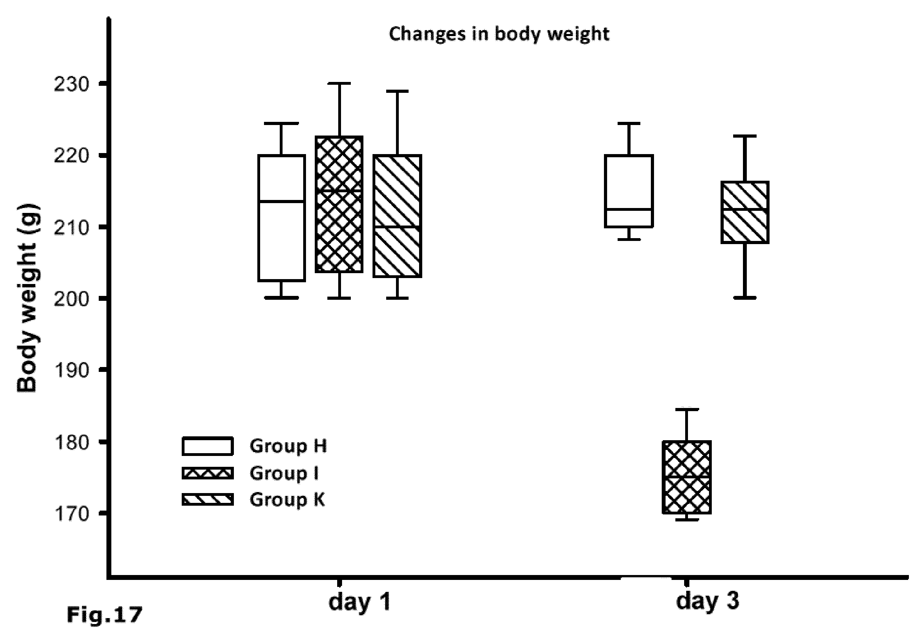

Figure 17 shows the change in body weight of the test animals with gastric injury without inflammation before and after the administration of ASS (group I) and the compound according to the invention (group K). Group H served as sham-operated controls. As can be seen from the data shown in Figure 17 the test animals suffered a severe loss of body weight within one day when administered ASS. In comparison, animals which were treated with the compound according to the invention (group K) only showed a minor loss in body weight compared to the animals that did not receive any pharmaceutically active compound. Further, the data clearly show that the compound of the invention does not cause additional stress and shows no adverse effects that would be expressed by loss of body weight.

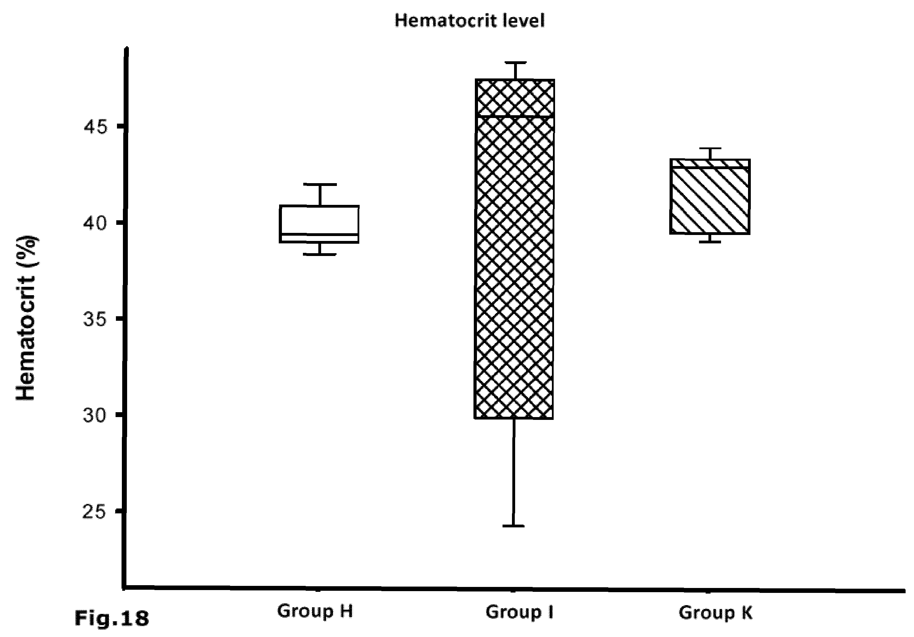

Figure 18 shows the percentage of haematocrit in the blood of the test animals after the animals were treated with the ASS and the compound according to the invention, respectively. No change in the haematocrit level in the blood of the animals treated with the compound according to the invention (group K) could be detected when compared with the control group (group H). On the other hand, animals of group I, treated with ASS shows a severe change in the haematocrit level.

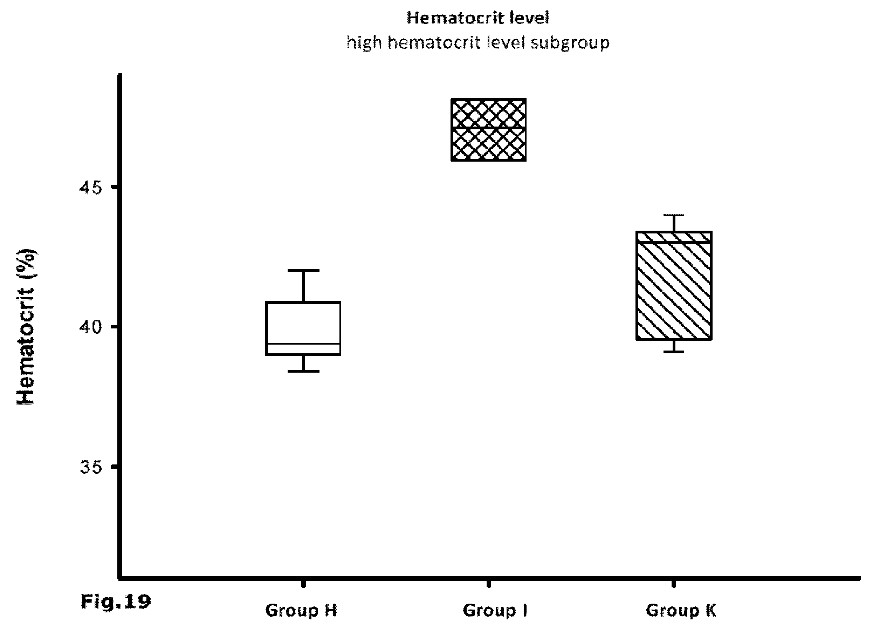

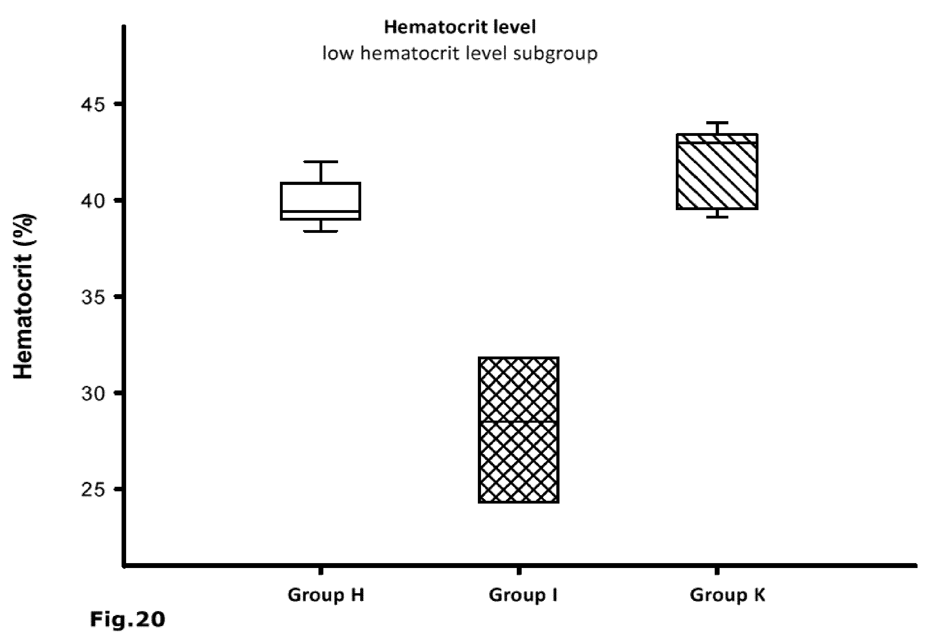

On the basis of the haematocrit measurements, the data can be divided into two groups:
a) high haematocrit level, indicating bleeding with plasma loss (Figure 19); and
b) low haematocrit level, indicating severe bleeding with plasma and cell loss (Figure 20).

As shown in Figure 19, test animals which were administered ASS showed an increase of the haematocrit level in the blood, indicating increased bleeding with plasma loss (group I), while no change in the haematocrit level could be detected in the animals of group K which were treated with the compound according to the invention instead when compared to the sham-operated group H.

As can be seen from the data shown in Figure 20, the haematocrit level of the animals of group I which received ASS shows a severe drop when compared to the control group H, indicating severe bleeding with loss of plasma and cells. In comparison, no changes could be detected in the animals of group K, treated with the compound according to the invention.

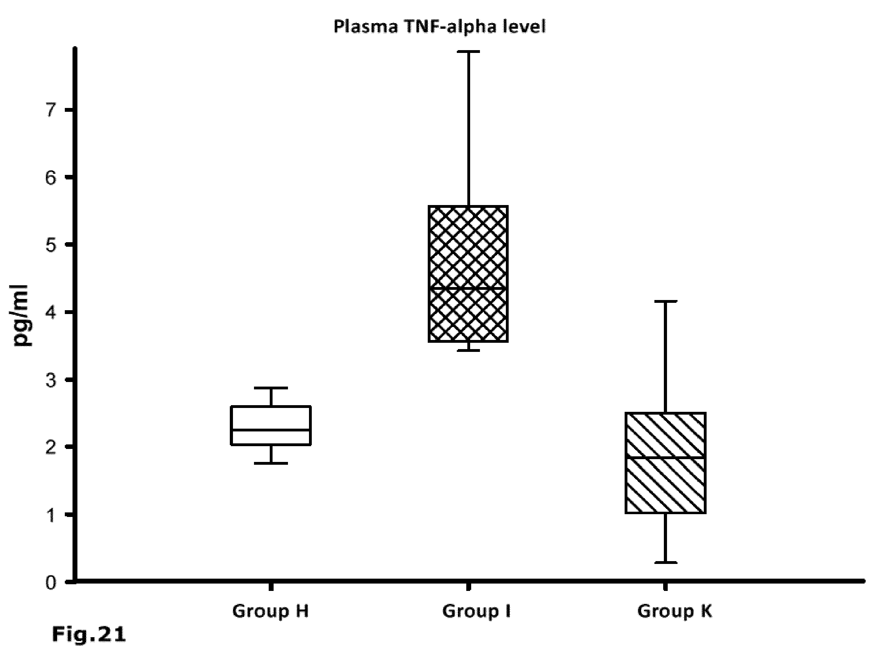

Figure 21 shows the results of the determination of the plasma TNF-α level of the test animals one day after administration of ASS and the compound according to the invention, respectively. Again, almost no change could be detected in case of the animals of group K, having been administered the compound according to the invention in comparison to the control group H. However, the animals of group I which were treated with ASS showed an increase in the plasma TNF-α level, indicating an increased number of macrophages in addition to those due to the gastric injury.

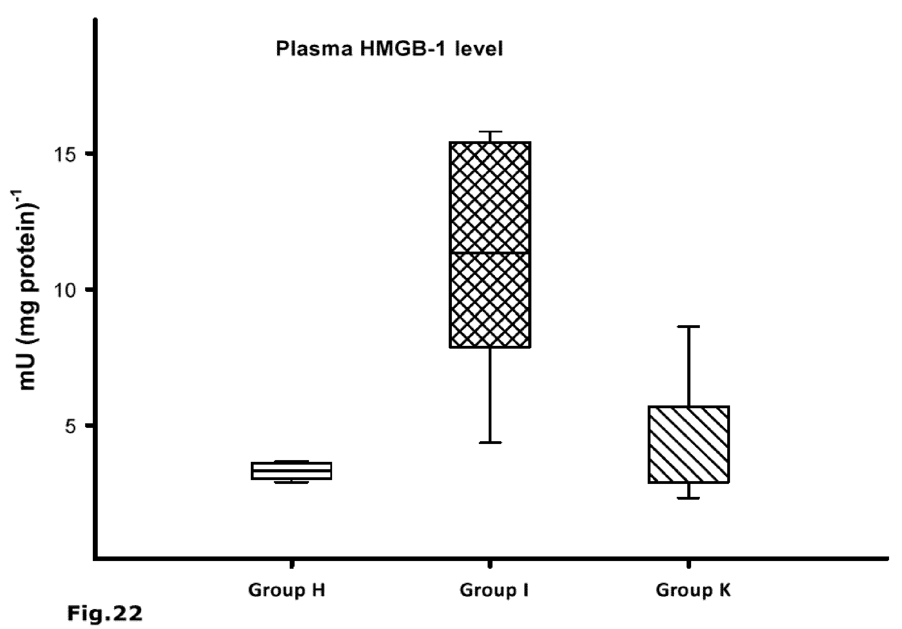

Figure 22 shows the concentration of the high mobility group protein B1 in the plasma of rats in group H, I and K on day 3. The concentration in the plasma of the animals in group K is comparable with the concentration in the plasma of the animals in the control group H. However, the concentration in the plasma of the animals of group I is increased.

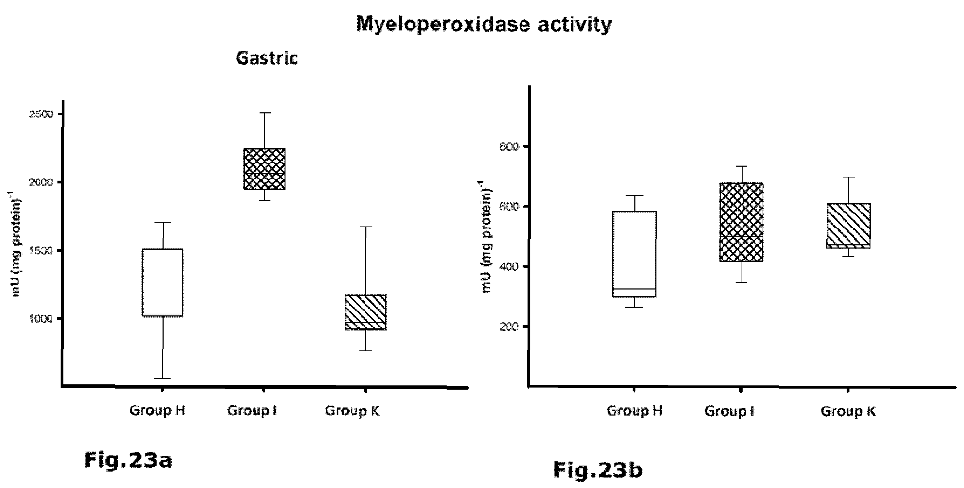

Figures 23a and 23b show the results of the determination of myeloperoxidase (MPO) activity in the gastric tissue (Figure 23a) and the colon tissue (Figure 23b) one day after the treatment of the test animals. As can be seen, the MPO activity of the animals treated with ASS (group I) is increased in comparison to those of the control group (group H) and those which were treated with the compound according to the invention (group K). Since MPO is an enzyme associated with inflammatory processes, it can be concluded that the treatment of rats with gastric injury with the compound according to the invention does not further enhance the inflammatory processes of the body, as is the case with ASS.

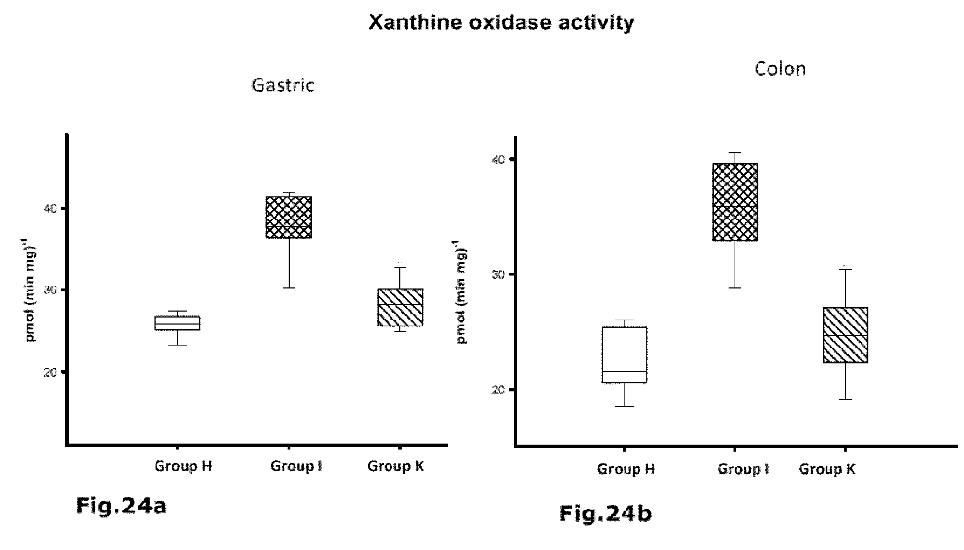

Figures 24a and 24b show the results obtained by determining the activity of xanthine oxidase enzyme in gastric and colon tissue (Figures 24a and 24b, respectively). Whereas animals treated with ASS showed an increased activity (group I), no such increase could be determined by the animals treated with the compound according to the invention (group K) in comparison to the control animals of group H. A high activity of xanthine oxidase enzyme may lead to an increased production of urea, which is associated with gout, and liver damage.

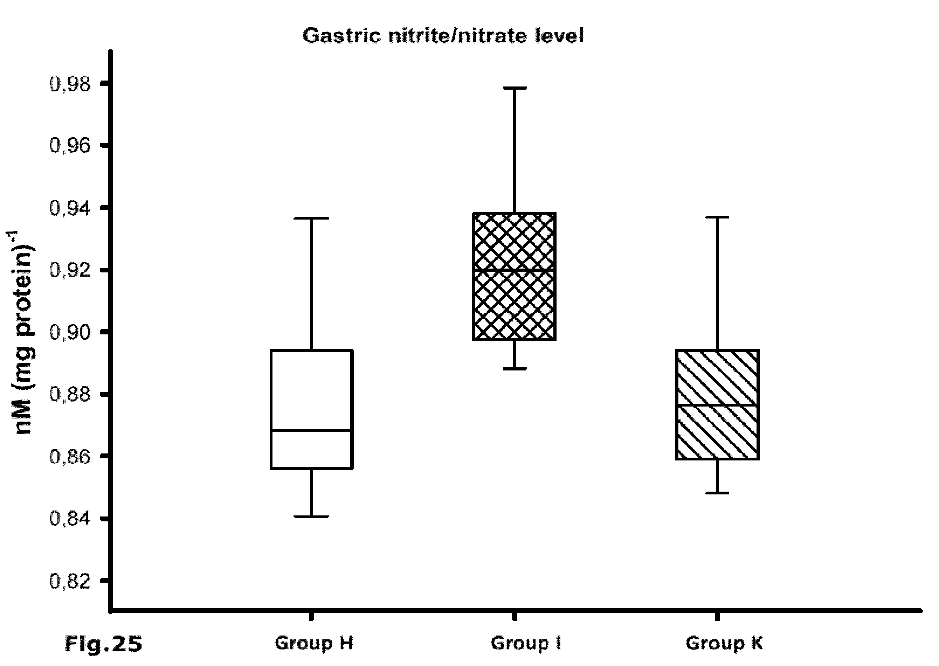

Figure 25 shows the results of the measurement of the nitrite/nitrate level in the blood of the test animals. Again, animals treated with ASS (group I) show an increased nitrite/nitrate level in comparison to animals of the control group H or animals of group I which were treated with the compound according to the invention.

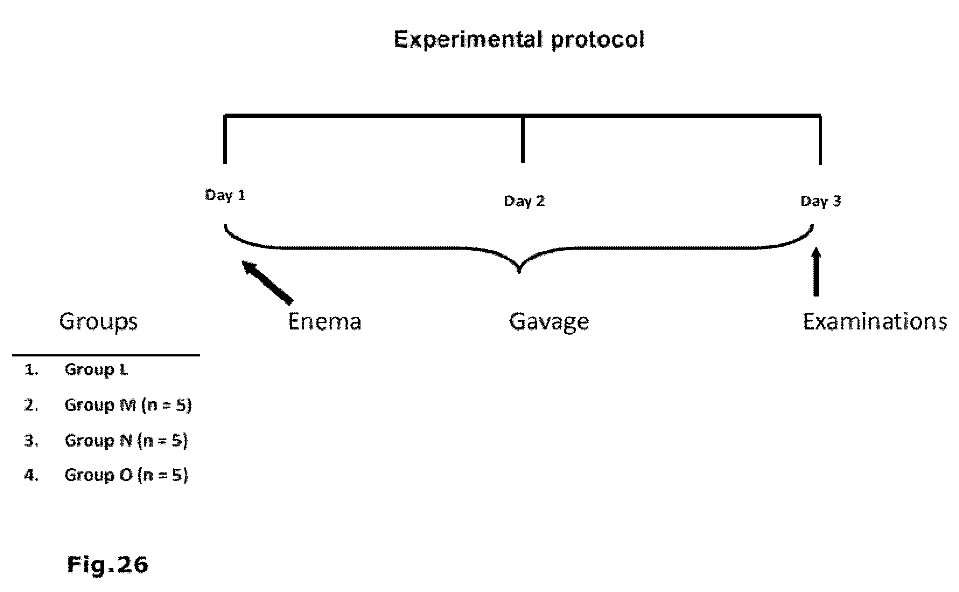

### Experimental Protocol (Figure 26)

The above-described clinical studies were repeated with animals having colitis which was induced as described above. 20 animals (rats) were randomly divided into four groups, wherein group L served as sham-operated control, having no colitis, group M served as second control having colitis but receiving no further treatment, group N having colitis and being treated with 1.11 mmol/kg body weight of ASS and group O having colitis and being treated with 1.11 mmol/kg of the compound according to the invention. One day after the animals received the enema with TNBS (40 mg/kg i.c.), wherein the animals of group L only received the respective solvent, the animals of group N and O were treated with ASS and the compound according to the invention, respectively. The other animals only received the respective solvent again. Two days after the enema the effects of the treatment were determined by measurement of body weight, blood gas analysis and biopsy. The animals were anesthetized two days after colitis induction. Surgery was performed to allow registration of the hemodynamic parameters at 1h intervals for 6 h (cardiac output [CO] data were measured only at the end of the experiments). Intravital videomicroscopy was performed at the end of the experiments to visualize the serosal microcirculation 3 cm distal from the cecum. In addition, fluorescence confocal endomicroscopy was performed to examine the microvasculature and morphologic changes of the mucosa of the distal colon, and full-thickness tissue samples and venous blood samples were taken to determine the biochemical changes in the colon and plasma.

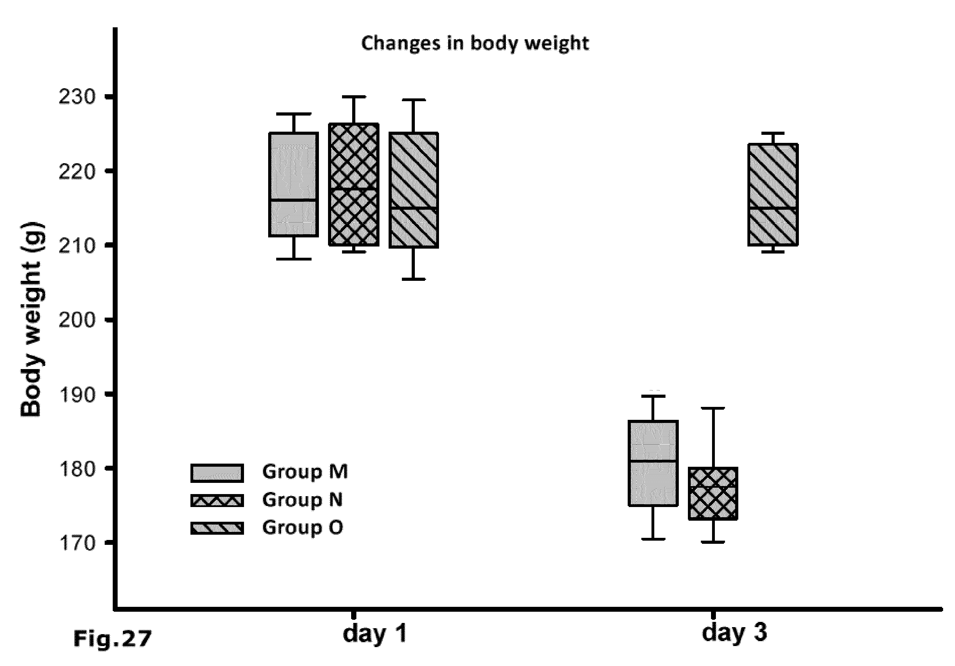

Figure 27 shows the loss of body weight the test animals suffered during the clinical tests. While the animals of groups M, N and O all show similar body weights the day of the enema, the animals of group M and N experienced a severe loss in body weight two days after the enema and one day after administration of ASS (group N). No weight loss was observed in case of the animals treated with the compound according to the invention (group O).

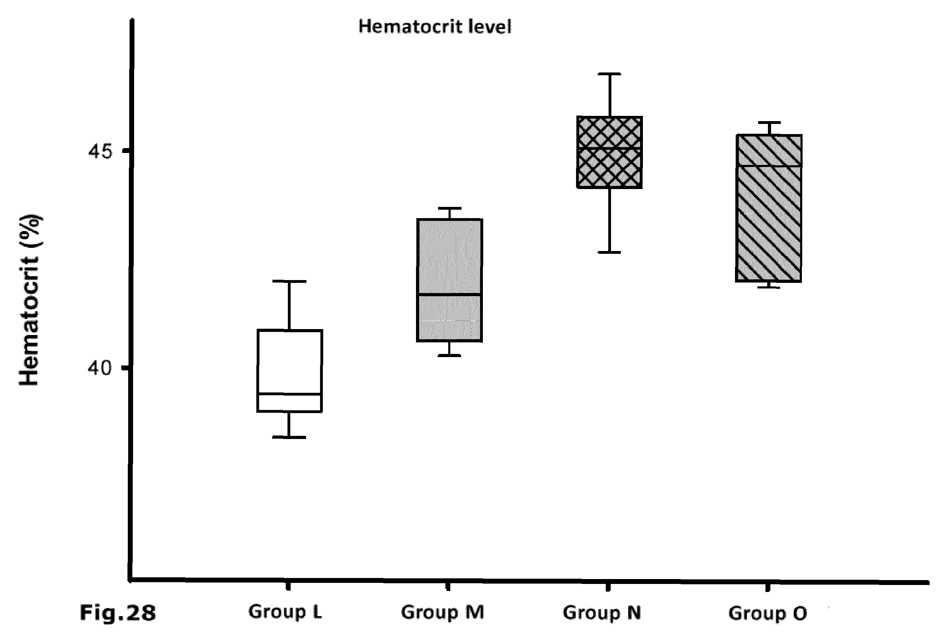

Figure 28 depicts the data received from the measurement of the haematocrit level in the blood of the test animals. As can be seen, animals treated with ASS showed an even higher level (group N) than those having colitis but receiving no further treatment (group M). The haematocrit level of the animals of group D, being treated with the compound according to the invention, was slightly higher than those of groups L and M.

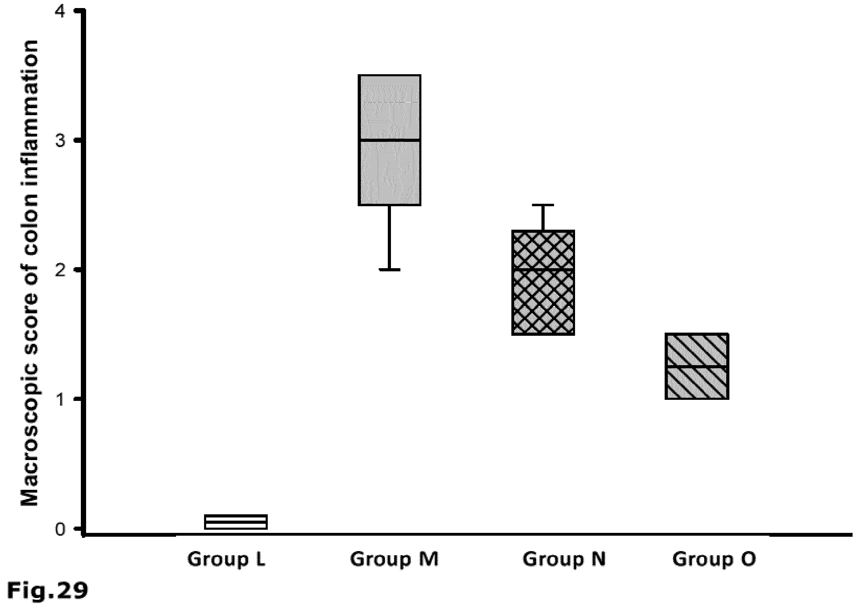

Figure 29 shows the macroscopic score of colon inflammation of the test animals. The score system is based on the work of Morris et al as described in the journal Gastroenterology 96:795-803, 1989 ("Hapten-induced model of chronic inflammation and ulceration in the rat colon"). The results are classified as follows:
0 = no signs of inflammation
1 = erythema
2 = erythema, edema and erosions
3 = two or more bleeding ulcers, inflammation and adhesions,
4 = severe ulceration and/or stenosis with dilatation of the proximal intestine.

As can be expected, animals of control group L did not show any signs of inflammation, wherein the animals of group M showed symptoms ranging from class 3 to 4. Animals of group N, treated with ASS, showed slightly less severe symptoms, whereas the animals of group O, treated with the compound according to the invention only showed increased erythema.

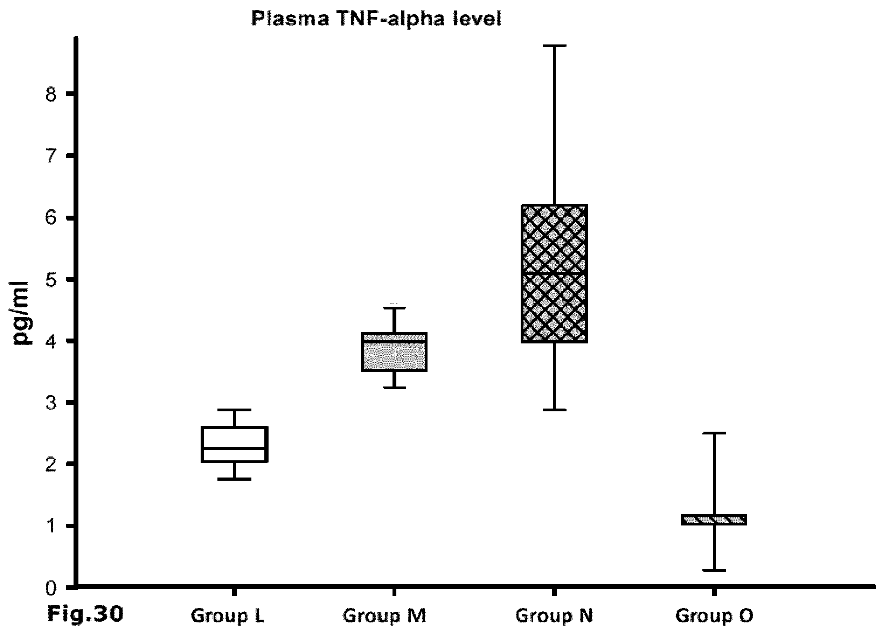

The data in Figure 30 shows the results of plasma TNF-α level determination of the test animals. As can be clearly seen, animals of group O, having been treated with the compound according to the invention, show a significant decrease in the TNF-α level, indicating that the increase in TNF-α caused by the induced colitis (group M) could be relieved by the administration of the compound according to the invention.

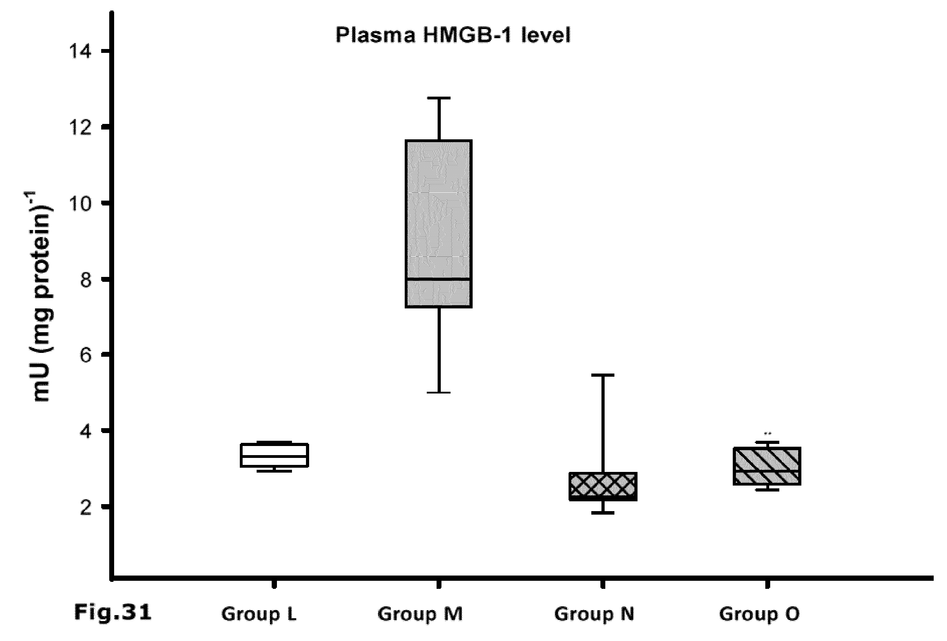

Figure 31 shows the concentration of the high-mobility group protein B1 in the plasma of the animals of group L, M, N and O on day 3. A significant reduction can be achieved with the treatment of the compound of the invention and shown for group O.

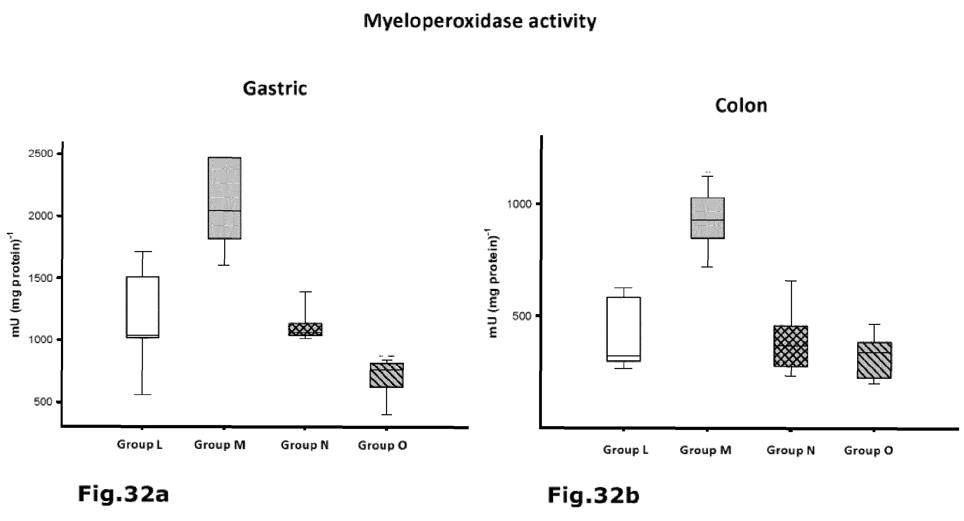

Figures 32a and 32b depict the myeloperoxidase enzyme activity of gastric tissue (Figure 32a) and colon tissue (Figure 32b) determined 3 days after induction of the colitis and one day after treatment. Again, the positive effect of the compound according to the invention can be seen, resulting in a decrease of activity when compared to animals having colitis without further treatment (group M) and those having colitis and having been treated with ASS (group N).

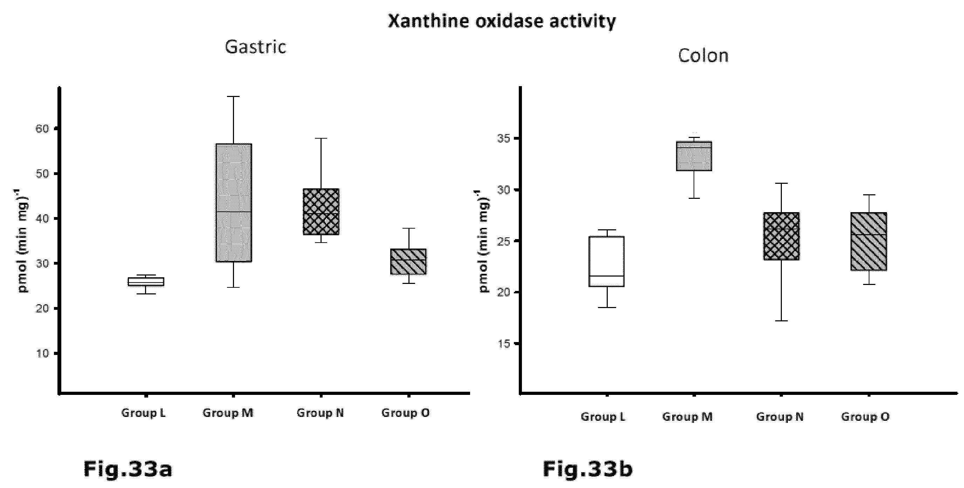

Figures 33a and 33b show the results obtained by determining the activity of xanthine oxidase enzyme in gastric and colon tissue (Figures 33a and 33b, respectively). Whereas animals treated with ASS (group N) show only a slight decrease in activity when compared to those receiving no medicinal treatment (group M). Animals of group O, having been treated with the compound according to the invention, showed an enzyme activity comparable to that of healthy animals, represented by group L, in both cases.

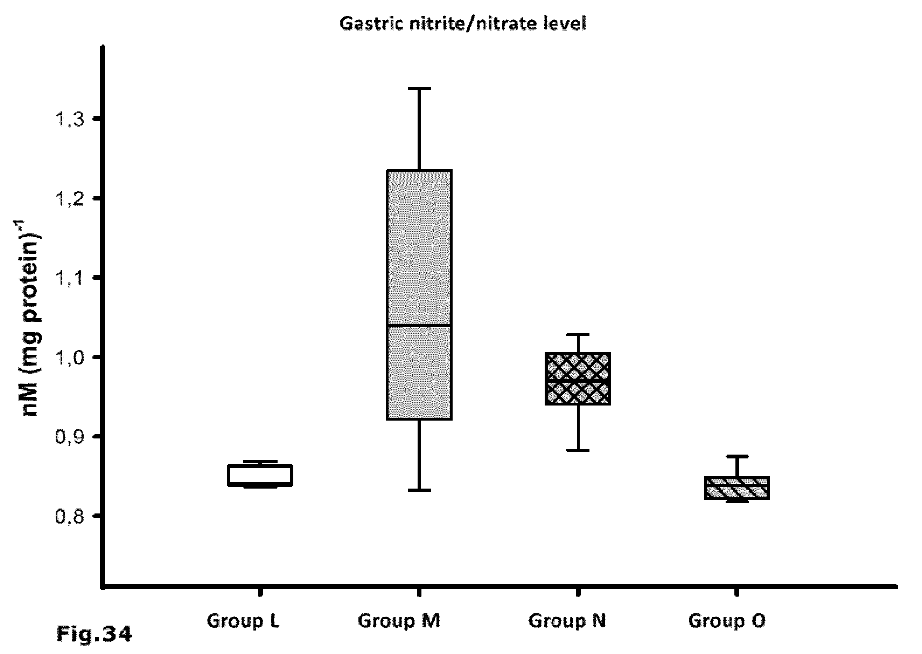

Figure 34 shows the results of the measurement of the nitrite/nitrate level in the blood of the test animals. Again, animals treated with ASS (group C) show an increased nitrite/nitrate level in comparison to animals of the control group L or animals of group M. Animals treated with the compound according to the invention on the other hand show nitrite/nitrate levels comparable to those of healthy animals despite having colitis.

As can be seen from Figures 17 to 34 on a rat model, the compound according to the invention shows a high medicinal activity, especially as TNF-α inhibitor and in the treatment of inflammatory bowel diseases such as colitis. Further, test animals treated with the compound of formula (I) did not suffer the adverse effects observed in the treatment with ASS. Additionally, the high-mobility group protein B1 concentration can be decreased as well as the peroxidase level.

Further, the damage of the mucosa can be reduced.

## Claims

1. Compound of formula (I):

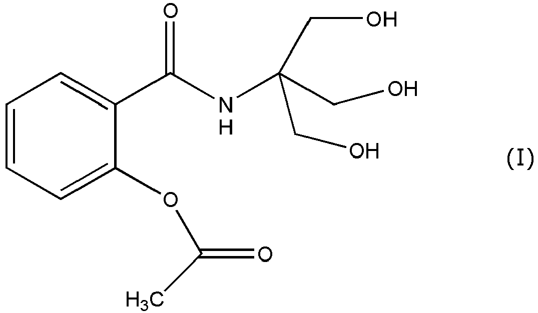

2. Anti-inflammatory agent of a compound of formula (I) as defined in claim 1.

3. Pharmaceutical composition comprising a compound of formula (I)

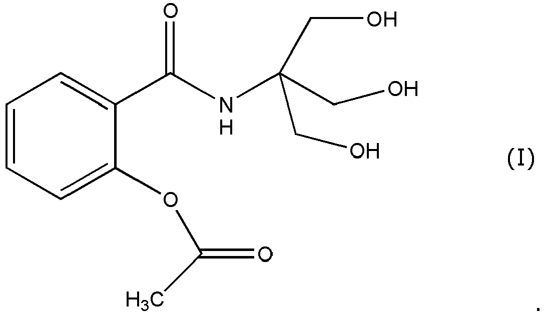

4. Pharmaceutical composition according to claim 3 comprising the compound of formula (I) in a pharmaceutically effective amount, preferably in an amount ranging from 0.001 to 10% by weight based on the total weight of the pharmaceutical composition.

5. Pharmaceutical composition according to claims 3 or 4 for use in the treatment and prophylaxis of inflammation.

6. Pharmaceutical composition according to at least one of claims 3 to 5, wherein the compound of formula (I) is administered in a dosage ranging from 0.001 to 0.3 g per kg body weight and day.

7. Pharmaceutical composition according to at least one of claims 3 to 6 for use in the treatment or prophylaxis of diseases selected from the group consisting of
a) psoriasis,
b) Crohn's disease,
c) ankylosing spondylitis,
d) psoriatic arthritis,
e) rheumatoid arthritis; and
f) ulcerative colitis.

8. Pharmaceutical composition according to claim 3 for use in the treatment of inflammatory bowel diseases.

9. Pharmaceutical composition according to at least one of claims 3 to 8 wherein the pharmaceutical composition is administered orally and/or parenterally.

10. Method for the preparation of a compound according to claim 1 comprising the following step:
reacting a compound of formula (II) 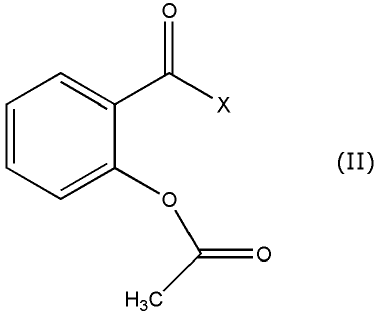
wherein X is a leaving group;
with a compound of formula (III) 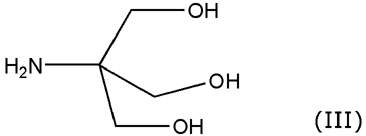 under formation of a compound according to formula (I).

11. Method according to claim 10 wherein X is selected from the group consisting of Halogen, -OH and -N₃.

12. Method according to claim 10 comprising the following steps:
i) providing a compound of formula (IV) 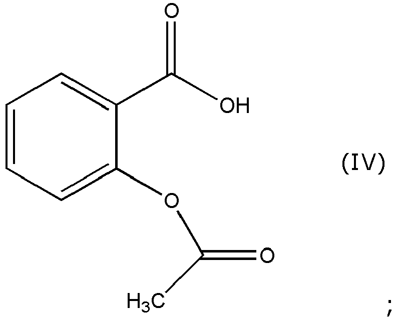
ii) activating the acid group of compound of formula (IV);
iii) converting the activated form of the compound of formula (IV) with a compound of formula (III) 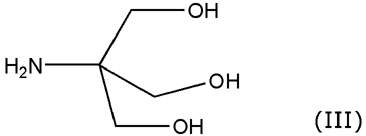 under formation of a compound of formula (I).

13. Method according to claim 12 wherein the steps i) to iii) are carried out in an organic solvent, preferably an aprotic organic solvent which is essentially free of water.

14. Method according to one or both of claims 12 and 13 wherein step ii) is carried out at a temperature ranging from -25 °C to -5 °C, preferably ranging from -20 °C to -10 °C.

15. Method according to at least one of claims 12 to 14 wherein step iii) is carried out at a temperature ranging from -25 °C to 30 °C, preferably ranging from -15 °C to 25 °C.

## Patentansprüche

1. Verbindung der Formel (I):

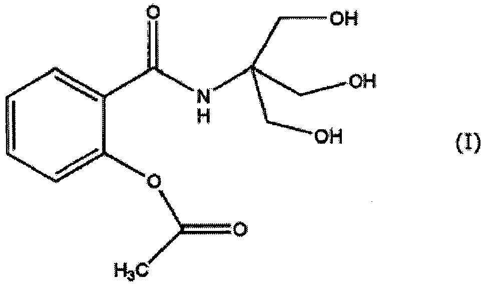

2. Entzündungshemmendes Mittel in Form einer Verbindung der Formel (I) gemäß Anspruch 1.

3. Pharmazeutische Zusammensetzung, die eine Verbindung der Formel (I) umfasst:

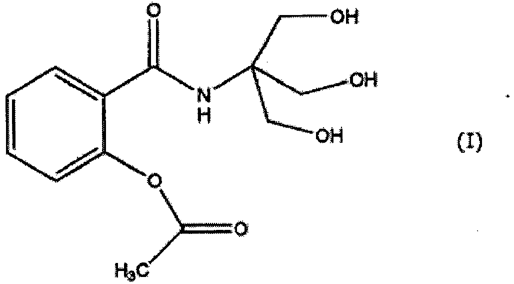

4. Pharmazeutische Zusammensetzung gemäß Anspruch 3, die die Verbindung der Formel (I) in einer pharmazeutisch wirksamen Menge umfasst, vorzugsweise in einer Menge im Bereich von 0,001 bis 10 Gew.-%, bezogen auf das Gesamtgewicht der pharmazeutischen Zusammensetzung.

5. Pharmazeutische Zusammensetzung gemäß Anspruch 3 oder 4 zur Verwendung bei der Behandlung und Prophylaxe von Entzündungen.

6. Pharmazeutische Zusammensetzung gemäß wenigstens einem der Ansprüche 3 bis 5, wobei die Verbindung der Formel (I) in einer Dosierung im Bereich von 0,001 bis 0,3 g pro kg Körpergewicht pro Tag verabreicht wird.

7. Pharmazeutische Zusammensetzung gemäß wenigstens einem der Ansprüche 3 bis 6 zur Verwendung bei der Behandlung oder Prophylaxe von Krankheiten, die aus der Gruppe ausgewählt sind, die aus
a) Psoriasis,
b) Morbus Crohn,
c) Spondylitis ankylosa,
d) Psoriasisarthritis,
e) rheumatoider Arthritis und
f) Colitis ulcerosa
besteht.

8. Pharmazeutische Zusammensetzung gemäß Anspruch 3 zur Verwendung bei der Behandlung von entzündlichen Darmerkrankungen.

9. Pharmazeutische Zusammensetzung gemäß wenigstens einem der Ansprüche 3 bis 8, wobei die pharmazeutische Zusammensetzung oral und/oder parenteral verabreicht wird.

10. Verfahren zur Herstellung einer Verbindung gemäß Anspruch 1, das den folgenden Schritt umfasst:
Umsetzen einer Verbindung der Formel (II) 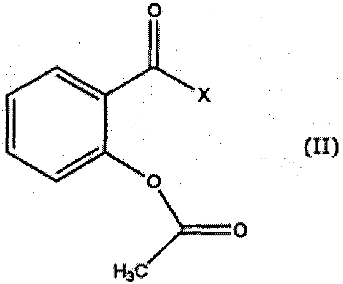
wobei X eine Abgangsgruppe ist,
mit einer Verbindung der Formel (III) 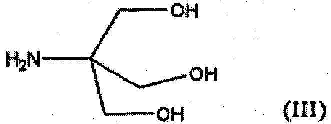 unter Bildung einer Verbindung gemäß Formel (I).

11. Verfahren gemäß Anspruch 10, wobei X aus der Gruppe ausgewählt ist, die aus Halogen, -OH und -N₃ besteht.

12. Verfahren gemäß Anspruch 10, das die folgenden Schritte umfasst:
i) Bereitstellen einer Verbindung der Formel (IV) 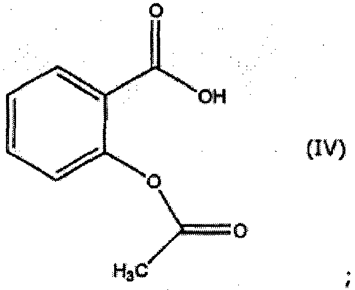
ii) Aktivieren der Säuregruppe der Verbindung der Formel (IV);
iii) Umsetzen der aktivierten Form der Verbindung der Formel (IV) mit einer Verbindung der Formel (III) 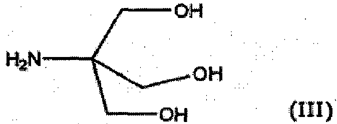 unter Bildung einer Verbindung der Formel (I).

13. Verfahren gemäß Anspruch 12, wobei die Schritte i) bis iii) in einem organischen Lösungsmittel, vorzugsweise einem aprotischen organischen Lösungsmittel, das im Wesentlichen frei von Wasser ist, durchgeführt werden.

14. Verfahren gemäß einem oder beiden Ansprüchen 12 und 13, wobei Schritt ii) bei einer Temperatur im Bereich von -25 °C bis -5 °C, vorzugsweise im Bereich von -20 °C bis -10 °C, durchgeführt wird.

15. Verfahren gemäß wenigstens einem der Ansprüche 12 bis 14, wobei Schritt iii) bei einer Temperatur im Bereich von -25 °C bis 30 °C, vorzugsweise im Bereich von -15 °C bis 25 °C, durchgeführt wird.

## Revendications

1. Composé répondant à la formule (I) :

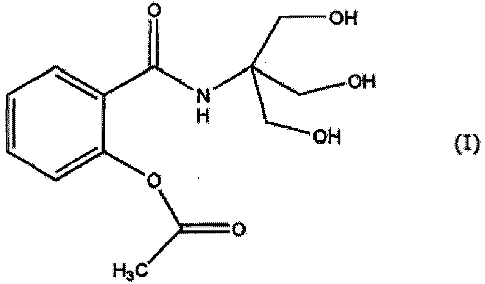

2. Agent anti-inflammatoire d'un composé répondant à la formule (I) tel que défini dans la revendication 1.

3. Composition pharmaceutique comprenant un composé répondant à la formule (I)

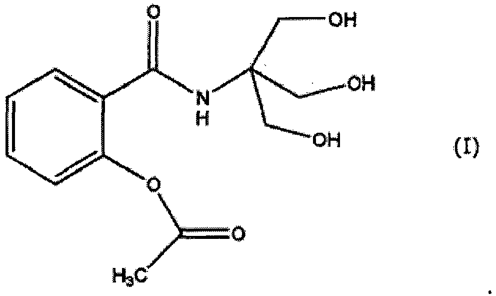

4. Composition pharmaceutique selon la revendication 3, comprenant le composé répondant à la formule (I) en une quantité pharmaceutiquement effective, de préférence en une quantité comprise entre 0,001 et 10 % en poids, par rapport au poids total de la composition pharmaceutique.

5. Composition pharmaceutique selon les revendications 3 ou 4 destinée à être utilisée dans le traitement et la prévention de l'inflammation.

6. Composition pharmaceutique selon l'une au moins des revendications 3 à 5, dans laquelle le composé répondant à la formule (I) est administré à un dosage compris entre 0,001 et 0,3 g par kg de poids corporel par jour.

7. Composition pharmaceutique selon l'une au moins des revendications 3 à 6 destinée à être utilisée dans le traitement et la prévention de maladies choisies dans le groupe consistant en
a) le psoriasis,
b) la maladie de Crohn,
c) la spondylarthrite ankylosante,
d) l'arthrite psoriasique,
e) la polyarthrite rhumatoïde, et
f) la colite ulcéreuse.

8. Composition pharmaceutique selon la revendication 3 destinée à être utilisée dans le traitement de maladies inflammatoires chroniques de l'intestin.

9. Composition pharmaceutique selon l'une au moins des revendications 3 à 8, dans laquelle ladite composition pharmaceutique est administrée par voie orale et/ou parentérale.

10. Procédé pour la préparation d'un composé selon la revendication 1, comprenant l'étape consistant à
faire réagir un composé de la formule (II) 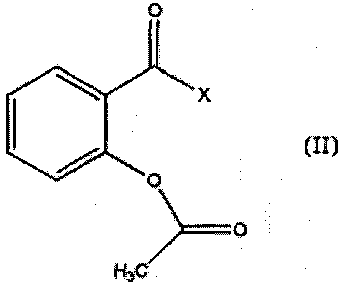 où X est un groupe partant,
avec un composé de la formule (III) 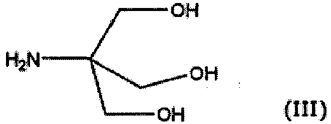 pour former un composé selon la formule (I).

11. Procédé selon la revendication 10, dans lequel X est choisi dans le groupe consistant en halogène, -OH et -N₃.

12. Procédé selon la revendication 10, comprenant les étapes consistant à
i) procurer un composé de la formule (IV) 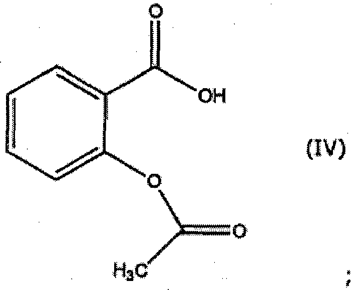
ii) activer le groupe acide du composé de la formule (IV),
iii) faire réagir la forme activée du composé de la formule (IV) avec un composé de la formule (III) 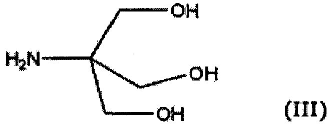 pour former un composé de la formule (I).

13. Procédé selon la revendication 12, dans lequel les étapes i) à iii) sont réalisées dans un solvant organique, de préférence un solvant organique aprotique qui est sensiblement exempt d'eau.

14. Procédé selon l'une ou les deux des revendications 12 et 13, dans lequel l'étape ii) est réalisée à une température comprise entre -25 °C et -5 °C, de préférence comprise entre -20 °C et -10 °C.

15. Procédé selon l'une au moins des revendications 12 à 14, dans lequel l'étape iii) est réalisée à une température comprise entre -25 °C et 30 °C, de préférence comprise entre -15 °C et 25 °C.
